# EUROPÄISCHE PATENTANMELDUNG

(11) **EP 0 585 812 A2**
(43) Veröffentlichungstag der Anmeldung: **09.03.1994**
(21) Anmeldenummer: 93113649.3
(22) Anmeldetag: 26.08.1993
(51) Int. Cl.: C08G 63/68, C07C 217/50, C08G 63/78, B01F 17/00, C09D 17/00, C07C 305/10, C07C 309/17, C08G 69/44, C08G 65/32, C09B 41/00, C09B 67/00, D06P 1/52, D06P 1/607, D21H 17/53, D21H 21/28

(54) **Grenzflächenaktive Verbindungen auf Basis alkoxylierter Fettamine**

(30) Priorität: 29.08.1992 DE 4228871
(71) Anmelder: HOECHST AKTIENGESELLSCHAFT, D-65929 Frankfurt am Main (DE)
(72) Erfinder: Uhrig, Heinz, D-61449 Steinbach/Taunus (DE); Münkel, Albert, D-65835 Liederbach (DE)

(57) **Zusammenfassung**

Die ständig spezifischer werdenden Anforderungen an die Eigenschaften von Pigmenten, besonders auf den Gebieten der Dispersions- und Druckfarben, machte die Entwicklung gezielter und umweltfreundlicher Dispergier-, Emulgier- und Kupplungshilfsmittel notwendig.

Die erfindungsgemäßen Verbindungen sind aus Fettamindialkylendiaminen hergestellte Oxalkylierungsprodukte, die gegebenenfalls durch Veresterung mit Dicarbonsäuren zu wiederkehrenden Struktureinheiten verknüpft sind, und worin vorzugsweise die Hydroxyendgruppen dieser Fettaminalkylendiamin-Oxalkylate mit Fettsäuren, aromatischen Carbonsäuren und/oder Harzsäuren verestert und noch vorhandene Hydroxygruppen mit Dicarbonsäuren und Sulfit zu den entsprechenden anionischen Resten aufweisenden Halbestern umgesetzt sind.

Die erfindungsgemäßen Verbindungen eignen sich für die verschiedensten Anwendungsbereiche auf dem Gebiet der grenzflächenaktiven Mittel, beispielsweise bei der Herstellung von Azopigmenten, Dispersions- und Druckfarben zur Verbesserung der coloristischen und rheologischen Eigenschaften. Die erfindungsgemäßen Verbindungen sind besonders umweltverträglich, da sie biologisch abbaubar sind.

## Beschreibung

Die Erfindung liegt auf dem Gebiet grenzflächenaktiver Mittel.

Bei der Herstellung von Dispersionen und Präparationen von Farbmitteln, beispielsweise Dispersionsfarbstoffen, besonders von anorganischen und organischen Pigmenten, werden für Anwendungen in wäßrigen und organischen Medien eine Vielzahl nichtionischer, anionischer und auch kationischer Tenside benutzt. Die Art der Tenside hat einen wesentlichen Einfluß auf die Feinverteilung und damit auf die Farbstärke der Farbmittel in den jeweiligen Anwendungsmedien. Außerdem werden Viskosität, Glanz und Lagerstabilität maßgeblich von der Art der Tenside beeinflußt. Auch bei der Herstellung von Azopigmenten und Azofarbstoffen werden Tenside üblicherweise auch zum besseren Ablauf der Kupplungsreaktion sowie zur Präparierung der Farbmittel benutzt. Die ständig steigenden Anforderungen an die coloristischen und rheologischen Eigenschaften der Azopigmente machen besonders auf dem Gebiet der Druckfarben eine gezielte Entwicklung von Präparationsmitteln notwendig, die die Fließfähigkeit von Druckfarben verbessern.

In EP-A-25 998 (US-PS 4 341 716) und EP-A-42 355 (US-PS 4 385 901) werden zur Herstellung gut fließfähiger Pigmentdispersionen und zur Herstellung von leicht dispergierbaren Farbstoffpräparationen Polyetherpolyamine und Dialkylsulfosuccinate sowie Schwefelsäureester auf Basis von oxalkylierten Fettaminen mit 12 bis 22 Kohlenstoffatomen im Fettrest beschrieben.

In der EP-A-195 328 (US-PS 4 705 889) und EP-A-197 005 (US-PS 4 713 482) werden Bis-(ω-aminoalkyl)-alkylamine mit Maleinsäure zum Halbamid umgesetzt und als Neutralsalz zum Emulgieren bei der Herstellung von Polymerdispersionen verwendet. Maleinsäure- und Phthalsäurehalbester von alkoxylierten Fettaminen werden zum Färben von wollhaltigen Fasermaterialien eingesetzt.

In der EP-A-235 088 (US-PS 4 778 919) werden Maleinsäure- und Phthalsäurehalbester von alkoxylierten Fettaminen als Egalisiermittel eingesetzt.

Weiterhin werden in EP-A-335 157 (US-PS 4 929 309) und EP-A-335 158 (US-PS 5 082 527 und 5 093 470) stickstoffhaltige, wasserlösliche Polymere, die durch Umsetzung von Halogenhydrin-Gruppen enthaltenden Reaktionsprodukten mit anorganischen Basen und anschließende Umsetzung der entstehenden Epoxy-Verbindungen mit halogenfreien Sulfonsäuren zu Sulfonsäureestern erhältlich sind, für die Naßfestausrüstung von Papier eingesetzt.

In der DE-A-3 145 734 (US-PS 4 416 808) werden Bis-Betaine auf Basis von alkoxylierten Fettaminalkylendiaminen als kosmetische Reinigungsmittel eingesetzt.

Keines der in den vorstehend genannten Druckschriften beschriebenen Produkte ist jedoch geeignet, den Kupplungsverlauf und die damit verbundene Präparierung von Azofarbmitteln hinsichtlich der Fließfähigkeit von Druckfarben entscheidend zu verbessern sowie schwerlösliche oder unlösliche Pigmente fein zu verteilen und zu stabilisieren, ohne andere Parameter, wie Farbstärke, Glanz oder Farbton, nachteilig zu beeinflussen.

Der vorliegenden Erfindung lag die Aufgabe zugrunde, neue grenzflächenaktive Mittel zur Verfügung zu stellen, die zur Herstellung gut fließfähiger, mineralölverträglicher, farbstarker und flockungsbeständiger Feststoffdispersionen, vorzugsweise Farbmitteldispersionen, für Druckfarben, für die Papiermassefärbung sowie gegebenenfalls für den Außenanstrich geeignet und weitgehend frei von den vorstehend genannten Nachteilen sind.

Gegenstand der vorliegenden Erfindung sind neue Verbindungen der Formel (I) 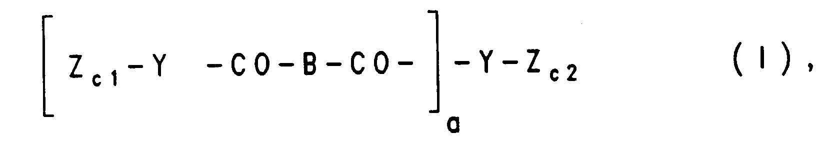
worin
- a: eine Zahl von 0 bis 9, vorzugsweise 1 bis 4, bedeutet;
- c1 und c2: gleich oder verschieden sind und eine Zahl von 4 bis 22, vorzugsweise 6 bis 12, bedeuten;
- Y: jeweils für gleiche oder verschiedene Einheiten der Formel (Ia) steht 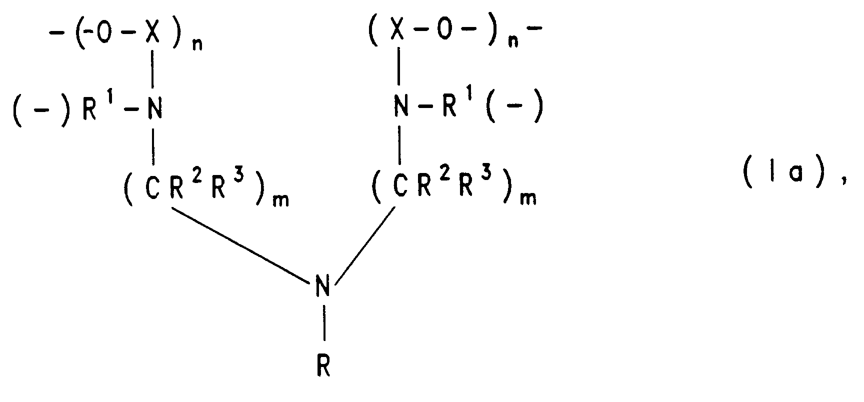
worin
- X: für eine Gruppe der Formeln -CH₂CH₂-, -CH(CH₃)CH₂- und -CH₂-CH(CH₃)- oder für eine Kombination davon steht;
- R: für einen gesättigten oder ungesättigten geradkettigen oder verzweigten C₁₂-C₂₂-Alkylrest,
- R¹: für ein Wasserstoffatom oder eine divalente Gruppe der Formel -(X-O)ₙ- mit einer wie vorstehend definierten, durch (-) angedeuteten freien Valenz,
- R² und R³: gleich oder verschieden sind und ein Wasserstoffatom oder eine Methylgruppe, vorzugsweise ein Wasserstoffatom,
- m: eine Zahl von 2 bis 3, vorzugsweise 3, und
- n: gleiche oder verschiedene Zahlen von 1 bis 200, vorzugsweise 1 bis 50, insbesondere 5 bis 30, bedeuten;
- Z: jeweils für gleiche oder verschiedene Reste Z¹ bis Z⁷ steht, worin
- Z¹: Wasserstoff,
- Z²: einen Acylrest einer geradkettigen, gesättigten oder ungesättigten C₂-C₂₂-Carbonsäure, vorzugsweise C₈-C₂₀-Carbonsäure, die unsubstituiert oder durch ein oder zwei Hydroxygruppen substituiert ist,
- Z³: einen Acylrest einer Di- oder Tricarbonsäure auf Basis einer di- oder trimerisierten C₈-C₂₄-Fettsäure,
- Z⁴: einen Acylrest der Formel R⁴-CO-, in der R⁴ einen Phenyl-, Naphthyl-, Hydroxyphenyl- oder Hydroxynaphthylrest bedeutet,
- Z⁵: einen Acylrest einer unmodifizierten oder modifizierten natürlichen Harzsäure,
- Z⁶: gleiche oder verschiedene Reste der Formeln -CO-CH=CH-COOM, -CO-(CH₂)_{q}-COOM, -CO-CH₂-CH(SO₃M)-COOM, -CO-CH(SO₃M)-CH₂-COOM, -OC-C₆H₄-COOM, worin q für eine ganze Zahl von 0 bis 10, vorzugsweise 2 bis 4, steht, und
- Z⁷: -SO₃M bedeuten,
worin M für Wasserstoff; ein Alkalimetall; ein Äquivalent eines Erdalkalimetalls; einen Oxalkylrest der Formel (X-O-)ₙH; eine Ammoniumgruppe, die unsubstituiert oder durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 1 bis 150, vorzugsweise 5 bis 30, Ethylenoxid- oder Propylenoxideinheiten oder einer Kombination von Ethylenoxid- und Propylenoxideinheiten erhaltene Ammoniumgruppe; oder eine Gruppe der Formel (II) 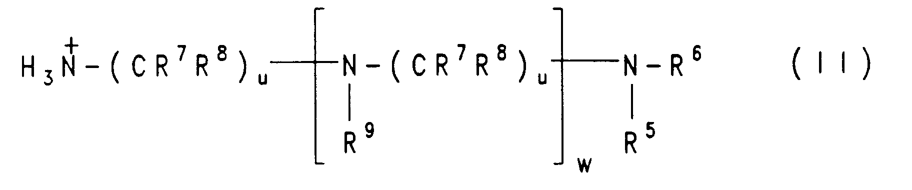
ist, worin R⁹, R⁵ und R⁶ gleich oder verschieden sind und für ein Wasserstoffatom oder eine Hydroxyalkylgruppe mit 1 bis 6 C-Atomen, vorzugsweise 2 bis 3 C-Atomen, und R⁷ und R⁸ gleich oder verschieden sind und für Wasserstoff oder Methyl stehen, u jeweils gleich oder verschieden ist und eine ganze Zahl von 2 bis 14, bevorzugt 2 bis 3, bedeutet und w für eine ganze Zahl von Null bis 25, bevorzugt von Null bis 5, steht; oder worin M für eine Gruppe der Formel (III) steht 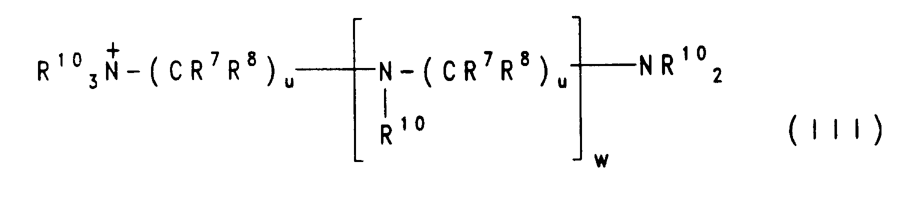
worin R¹⁰ die Gruppe H-(O-X-)_{y} bedeutet, worin X die vorstehend genannten Bedeutungen hat und y eine ganze Zahl von 1 bis 100, vorzugsweise 1 bis 30, ist, und
- B: für einen geradkettigen, verzweigten oder cyclischen aliphatischen Rest mit jeweils 1 bis 60 C-Atomen, vorzugsweise 1 bis 30 C-Atomen, insbesondere für geradkettiges C₁-C₈-Alkylen, für C₆-C₁₂-Arylen oder für eine Gruppe der Formeln -CH=CH-, -CH(SO₃M)CH₂- oder -CH₂CH(SO₃M)- steht, worin M für ein Kation oder für einen Rest der Formel -(X-O-)ₙH steht;
und wobei mindestens ein Rest Z ein Rest aus der Gruppe Z², Z³, Z⁴, Z⁵ und Z⁶ ist; oder wobei mindestens zwei Einheiten der Formel (Ia) über eine divalente Gruppe -CO-B-CO- miteinander verknüpft sind und Z die Bedeutung von Z¹ bis Z⁷ hat.

Von besonderem Interesse sind Verbindungen der Formel (I), worin
- a: eine Zahl von 1 bis 4 bedeutet;
- c1 und c2: gleich oder verschieden sind und eine Zahl von 6 bis 12 bedeuten.

Von besonderem Interesse sind Verbindungen der Formel (I), in der
- R: für einen gesättigten oder ungesättigten geradkettigen oder verzweigten C₁₂-C₁₈-Alkylrest;
- X: zu 50 bis 100 %, vorzugsweise zu 80 bis 100 %, aller Reste X für eine Gruppe der Formel -CH₂CH₂- und zu 0 bis 50 %, vorzugsweise zu 0 bis 20 %, aller Reste X für eine Gruppe der Formeln -CH(CH₃)CH₂- oder -CH₂-CH(CH₃)-;
- B: für C₁-C₆-Alkylen, 1,2-, 1,3- oder 1,4-Phenylen, eine Gruppe der Formeln -CH=CH-, -CH(SO₃M)CH₂- oder -CH₂CH(SO₃M)-, worin M für ein Kation steht,
- m: für die Zahl 3 und
- n: für gleiche oder verschiedene Zahlen von 1 bis 50, vorzugsweise 5 bis 30, stehen.

Besonders bevorzugt sind Verbindungen der Formel (I), in denen 20 bis 100 %, vorzugsweise 30 bis 70 %, der Reste Z jeweils unabhängig voneinander die Bedeutung von Z², Z³, Z⁴, Z⁵, Z⁶ oder einer Kombination davon haben und 80 bis 0 %, vorzugsweise 70 bis 30 %, der Reste Z die Bedeutung von Z¹, Z², Z⁴, Z⁵ oder einer Kombination davon haben.

Von besonderem Interesse sind ferner Verbindungen der Formel (I), in der a eine Zahl von 1 bis 4 ist und Z die Bedeutung Z¹ hat.

Gegenstand der vorliegenden Erfindung ist auch ein Verfahren zur Herstellung einer erfindungsgemäßen Verbindung der Formel (I), dadurch gekennzeichnet, daß man
a) ein der Formel (Ia) zugrundeliegendes Alkylaminodialkylendiamin mit Ethylenoxid oder Propylenoxid oder beiden Epoxiden hintereinander oder einem Gemisch beider Epoxide oxalkyliert,
b) anschließend das in Schritt a) erhaltene Oxalkylat der Formel (Ia)
b1) mit mindestens einem der Acylreste Z², Z³, Z⁴, Z⁵, Z⁶ und -OC-B-CO-zugrundeliegenden Carbonsäure oder einem reaktiven Derivat dieser Carbonsäure, vorzugsweise einem Anhydrid, in einer oder mehreren Stufen vollständig verestert oder teilverestert oder mit mehreren der vorstehend genannten Carbonsäuren oder deren reaktiven Derivaten mischverestert,
b2) und vorhandene Maleinsäurehalbestergruppen gegebenenfalls mit einem Sulfit oder schwefliger Säure umsetzt,
b3) gegebenenfalls die nach a) erhaltenen Oxalkylate mit einem dem Rest Z⁷ zugrundeliegenden Sulfatierungsmittel, vorzugsweise Schwefelsäure, Chlorsulfonsäure, Amidosulfonsäure oder Schwefeltrioxid, sulfatiert; und
c) im Falle, daß in b) ein Rest der Formel Z⁶ oder Z⁷ eingeführt wurde, gegebenenfalls mit einer dem Rest M zugrundeliegenden Base in das entsprechende Salz oder Oxalkylat überführt.

Die der Formel (Ia) zugrundeliegenden Alkylaminodialkylendiamine werden beispielsweise erhalten, indem man ein primäres Amin der Formel R-NH₂ mit einem reaktionsfähigen Nitril von 2 bis 3 C-Atomen (einschließlich der CN-Gruppe) zu einer Verbindung der allgemeinen Formel (II) 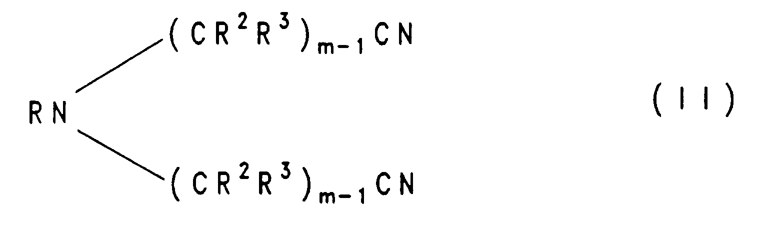
in einer Dicyanalkylierungsreaktion umgesetzt. Diese Reaktion ist beispielsweise aus der US-PS 3 028 415 bekannt. Sie kann sowohl mit saurer als auch mit basischer Katalyse, mit Hilfe von Lösungsmitteln wie Wasser oder auch niederkettigen Alkoholen drucklos oder unter erhöhtem Druck, kontinuierlich oder diskontinuierlich durchgeführt werden. Als saure Katalysatoren sind beispielsweise Essigsäure, Phosphorsäure, Salzsäure oder andere Mineralsäuren geeignet (US-PS 3 615 797, US-PS 3 028 415), als basische Katalysatoren sind beispielsweise Natrium- oder Kaliumhydroxid, Alkalialkoholate, Trimethylbenzylammoniumhydroxid oder Morpholin geeignet (Kirk-Othmer, Encyclopedia of Chemical Technology, 1965, Band 6, Seite 634 ff.; H.A. Bruson "Cyanoethylation", Organic Reactions 5, 1949, Seite 79 ff., Verlag John Wiley and Sons, New York). Als Co-Katalysatoren oder auch als Lösungsvermittler werden Wasser oder niedere Alkohole, wie Methanol, Ethanol, Isopropanol oder Gemische derselben, in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines inertgases, und bei Temperaturen von 60 bis 150°C durchgeführt. Das Cyanalkylierungsmittel, vorzugsweise Acrylnitril oder Chloracetonitril, wird stöchiometrisch oder in einem bis zu vierfachen Überschuß eingesetzt.

Anschließend wird das Dicyanalkylierungsprodukt der Formel (II) in Gegenwart von Wasserstoff zu einer Verbindung der Formel (III) 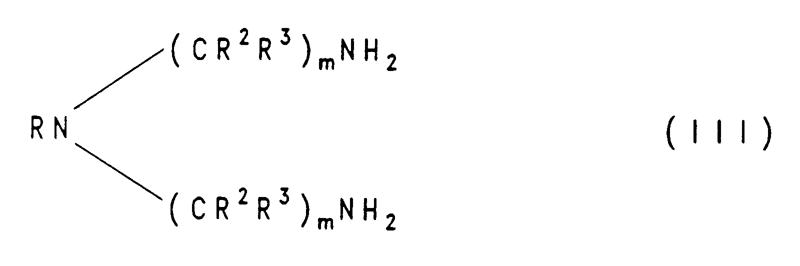
reduziert. Die Reduktion wird mit Raney-Nickel, Raney-Kobalt oder mit Nickel- oder Kobalt-Trägerkatalysatoren durchgeführt, und zwar unter Einsatz von 1 bis 10 Gew.-% Katalysator, bevorzugt 1 bis 5 Gew.-%, bezogen auf die Verbindung der Formel (III), und bei einem Druck im Bereich von 50 bis 200 bar Wasserstoff und Temperaturen von 60 bis 150°C; die Reaktionszeit beträgt dabei etwa 1 bis 5 Stunden.

Als Amine der Formel R-NH₂ sind beispielsweise von Interesse: Kokosfettamin, Palmkernfettamin, Talgfettamin, Stearylamin, Laurylamin, Oleylamin oder Gemische der genannten Fettamine.
a) Die Oxalkylierung der Fettaminalkylendiamine der Formel (III) kann nach üblichen Methoden erfolgen. Vorzugsweise wird das jeweilige Fettaminalkylendiamin bei einer Temperatur von 100 bis 200°C, vorzugsweise bei 120 bis 160°C, mit Ethylenoxid oder Propylenoxid oder beiden Epoxiden, abwechselnd oder als Gemisch, in Gegenwart eines Hydroxids oder eines Alkoxylats als Katalysator, vorzugsweise eines Alkalihydroxids, wie Kaliumhydroxid oder insbesondere Natriumhydroxid, oder eines Alkalialkoxylats, wie Natriummethylat oder Natriumethylat, umgesetzt. Die Menge der verwendeten Epoxide oder des Epoxidgemisches wird so bemessen, daß pro reaktionsfähigem Wasserstoffatom der freien Aminogruppen des jeweiligen Fettaminalkylendiamins 1 bis 200, vorzugsweise 1 bis 50, insbesondere 5 bis 30, Mol des oder der verwendeten Epoxide angelagert werden. Die Konzentration des Katalysators beträgt bevorzugt 0,05 bis 1,0 Gew.-%, bezogen auf das Fettaminalkylendiamin` bei Beginn der Oxalkylierung. Ein Katalysator ist nicht erforderlich, wenn vorzugsweise nur eine Ethylenoxid- oder Propylenoxid-Einheit pro Kette angelagert werden soll. Die Oxalkylierung kann drucklos oder in Druckgefäßen mit Propylenoxid oder mit Ethylenoxid oder mit Mischungen von beiden Epoxiden durchgeführt werden, wobei das Alkylenoxid gasförmig oder flüssig zugeführt werden kann. Der Arbeitsdruck beträgt in der Regel 1 bis 10 bar, vorzugsweise 2 bis 8 bar. Die Menge des angelagerten Alkylenoxids kann je nach dem beabsichtigten Einsatzzweck und dem angestrebten Grad der Hydrophilie der aus den Oxalkylierungsprodukten hergestellten Verbindungen der Formel (I) variiert und optimiert werden.
b) Die Veresterung der Fettaminalkylendiamin-Oxalkylate der Formel (Ia) erfolgt in ein oder mehreren Reaktionsstufen, wobei die Einführung der Reste Z mit der Bedeutung Z², Z³, Z⁴, Z⁵ und Z⁶ in der Regel durch Umsetzung mit entsprechenden Carbonsäuren, Dicarbonsäuren, deren Estern oder deren Anhydriden vorgenommen wird.
b1) Man kann auf diese Weise einen Teil oder alle Hydroxygruppen der Fettaminalkylendiamin-Oxalkylate mit den vorstehend genannten Carbonsäuren oder deren Derivaten verestern. Die Veresterung erfolgt im molaren Verhältnis Fettaminalkylendiamin-Oxalkylat:Carbonsäure(derivat) im Bereich von 1:1 bis 1:4. Es ist auch möglich, Gemische der genannten Carbonsäuren oder deren Derivaten einzusetzen. Meist ist es vorteilhaft, in einer ersten Reaktionsstufe einen Teil der vorhandenen Hydroxygruppen mit monofunktionellen Carbonsäuren zu verestern und danach in einer zweiten Reaktionsstufe mit Dicarbonsäuren zu verestern, wobei in der zweiten Reaktionsstufe je nach der eingesetzten Menge an Dicarbonsäure anionische Reste in Form freier Carboxylatgruppen eingeführt werden, oder, vor allem bei einem Unterschuß an Dicarbonsäure, eine Verknüpfung von 2 bis 10 Einheiten der Verbindungen der Formel (Ia) über einen zweiwertigen Rest -OC-B-CO- stattfindet.
   Die dem Acylrest Z² zugrundeliegenden Säuren sind beispielsweise Essigsäure, Propionsäure, Buttersäure, Valeriansäure, Capronsäure, Heptansäure, Octansäure, Pelargonsäure, Caprinsäure, Undecansäure, Laurinsäure, Tridecansäure, Myristinsäure, Pentadecansäure, Palmitinsäure, Heptadecansäure, Stearinsäure, Nonadecansäure, Arachinsäure, Behensäure, 10-Undecensäure, Lauroleinsäure, Myristoleinsäure, Palmitoleinsäure, 6c- und 6t-Octadecensäure, Elaidinsäure, Ölsäure, Linolsäure, Linolensäure, Rizinolsäure oder Rizinensäure, insbesondere geradzahlige Fettsäuren oder Hydroxyfettsäuren mit jeweils 8 bis 20 C-Atomen, beispielsweise die vorstehend genannten geradzahligen Fettsäuren sowie insbesondere ihre aus Naturprodukten gewonnenen Gemische, wie Tallölfettsäure, Talgfettsäure, Kokosölfettsäure, Palmölfettsäure, Leinölfettsäure, Rizinusölfettsäure und Rizinensäure, besonders bevorzugt die genannten Fettsäuren mit 12 bis 18 C-Atomen und deren Gemische.
   Die dem Acylrest Z³ zugrundeliegenden Di- oder Tricarbonsäuren sind dimerisierte oder trimerisierte Fettsäuren mit 28 bis 72, insbesondere 36 bis 54, Kohlenstoffatomen.
   Die dem Acylrest Z⁴ zugrundeliegenden Säuren oder Anhydride sind beispielsweise Benzoesäure, Salicylsäure, o-, m- und p-Methylbenzoesäuren, Naphthoesäuren, insbesondere Hydroxynaphthoesäuren, beispielsweise 3-Hydroxy-1-naphthoesäure, 3-Hydroxy-2-naphthoesäure, 4-Hydroxy-2-naphthoesäure, 5-Hydroxy-1-naphthoesäure, 5-Hydroxy-2-naphthoesäure, 6-Hydroxy-2-naphthoesäure und 7-Hydroxy-2-naphthoesäure.
   Die dem Acylrest Z⁵ zugrundeliegenden Harzsäuren sind unmodifizierte oder modifizierte Naturharzsäuren vom Kolophoniumtyp oder deren reaktionsfähige Derivate, vorzugsweise Harzsäuren wie Abietinsäure, Dehydroabietinsäure, Tetrahydroabietinsäure, Lävopimarsäure, Dextropimarsäure oder Isodextropimarsäure, wie sie in handelsüblichen Kolophoniumarten vorliegen sowie modifizierte Harzsäuren, wie disproportionierte, hydrierte und dimerisierte Naturharzsäuren.
   Die dem Acylrest Z⁶ zugrundeliegenden Säuren oder Carbonsäureanhydride sind beispielsweise Maleinsäure, Maleinsäureanhydrid, Fumarsäure, Phthalsäure, Phthalsäureanhydrid, Isophthalsäure, Terephthalsäure, Oxalsäure, Malonsäure, Bernsteinsäure, Bernsteinsäureanhydrid, Glutarsäure, Glutarsäureanhydrid, Adipinsäure, Pimelinsäure, Korksäure, Acelainsäure, Sebacinsäure und Sulfobernsteinsäure, vorzugsweise Bernsteinsäure, Phthalsäure, Terephthalsäure, Maleinsäure und Fumarsäure, insbesondere Sulfobernsteinsäure.
   Die Veresterung der Fettaminalkylendiamin-Oxalkylate der Formel (Ia) mit den vorstehend genannten Carbonsäuren oder Dicarbonsäuren sowie die Verknüpfung zwei oder mehrerer Fettaminalkylendiamin-Oxalkylate der Formel (Ia) kann nach an sich üblichen Veresterungsmethoden erfolgen. Die einzuhaltende Reaktionstemperatur liegt dabei in der Regel zwischen Raumtemperatur und 240°C, je nach Veresterungsmethode. Bevorzugt wird zur Erhöhung der Ausbeute die Veresterung in einem inerten organischen Lösungsmittel durchgeführt, das als Schleppmittel zum Entfernen des Reaktionswassers geeignet ist. Beispielsweise kann die Veresterung in Xylol als organischem Lösungsmittel und in Gegenwart von sauren Katalysatoren bei einer Temperatur von 130 bis 220°C durchgeführt werden. Als saure Katalysatoren sind beispielsweise Säuren und Lewissäuren, wie Benzolsulfonsäure, p-Toluolsulfonsäure, Borsäure, Zinnpulver, Zinkchlorid oder Schwefelsäure möglich. Zinnpulver wird in einer Menge von 1 bis 4 Gew.-%, die übrigen genannten Säuren werden in einer Menge von 0,1 bis 2 Gew.-%, vorzugsweise 0,5 bis 1 Gew.-%, jeweils bezogen auf die umzusetzende Verbindung, eingesetzt. Im Falle der Anhydride der genannten Säuren geschieht die Veresterung durch Mischen und Verrühren bei 10 bis 120°C, bevorzugt bei 40 bis 80°C, in Anwesenheit von Alkalihydroxiden. Die Konzentration der Alkalihydroxide beträgt zweckmäßigerweise 0,1 bis 1,0 Gew.-%, bezogen auf die Gesamtmischung. Die Einführung der anionischen Gruppen geschieht vorzugsweise durch Veresterung mit Maleinsäureanhydrid oder Phthalsäureanhydrid durch Mischen und Verrühren bei 20 bis 100°C, bevorzugt bei 40 bis 80°C, in Anwesenheit von Alkalihydroxiden. Die Konzentration der Alkalihydroxide beträgt vorzugsweise 0,1 bis 1,0 Gew.-%, bezogen auf die Gesamtmischung. Im Fall von Maleinsäureanhydrid ist es wegen der Sublimationsneigung vorteilhaft, in Druckgefäßen unter einem Überdruck von 0,2 bis 1 bar Stickstoff oder Luft zu arbeiten und für kräftiges Durchmischen zu sorgen, da zu Beginn der Reaktion das geschmolzene Maleinsäureanhydrid mit den teilveresterten Oxalkylaten schlecht mischbar ist.
   Die Veresterung der oxalkylierten Fettaminalkylendiamine mit den genannten Harzsäuren, Fettsäuren oder aromatischen Carbonsäuren kann alternativ auch durch Umesterung unter Einsatz der entsprechenden Alkylester, vorzugsweise Methylester, der genannten Säuren in Gegenwart von 0,1 bis 1,0 Mol-Äquivalenten Alkoxylat, vorzugsweise Natriummethylat, bei 150 bis 200°C, vorzugsweise 160 bis 190°C, unter Abdestillieren des frei werdenden Alkanols, vorzugsweise Methanols, erfolgen.
   Die erfindungsgemäßen nicht-ionogenen Harzsäure-, Fettsäure- oder aromatischen Carbonsäureester der Fettaminalkylendiamin-Oxalkylate sind als solche wertvolle oberflächenaktive Mittel und können im Sinne der Erfindung eingesetzt werden.
   Die Verknüpfung mehrerer Fettaminalkylendiamin-Einheiten der Formel (Ia) kann durch Umsetzung der Fettaminalkylendiamin-Oxalkylate in einer ersten Reaktionsstufe durch Veresterung mit einer oder mehreren Dicarbonsäuren der Formel HOOC-B-COOH im Molverhältnis von 2:1 bis 10:9, vorzugsweise 2:1 bis 5:4, erfolgen. Als Dicarbonsäuren eignen sich zur Verknüpfung vorzugsweise aliphatische Dicarbonsäuren mit 3 bis 32 Kohlenstoffatomen, insbesondere Malonsäure, Maleinsäure, Bernsteinsäure, Glutarsäure, Adipinsäure, 1,5-Pentandicarbonsäure, 1,6-Hexandicarbonsäure oder 1,10-Decandicarbonsäure, aber auch beispielsweise Cyclohexan-1,4-dicarbonsäure und aromatische Dicarbonsäuren, wie Phthalsäure oder Terephthalsäure. Anstelle der Carbonsäuren können zur Veresterung auch, soweit vorhanden, die Anhydride oder reaktive Carbonsäurederivate eingesetzt werden, beispielsweise im Falle von Umesterungsreaktionen niedere Alkylester der Carbonsäuren.
   Die verknüpften Fettaminalkylendiamin-Oxalkylateinheiten sind bereits oberflächenaktive Mittel und können im Sinne der Erfindung eingesetzt werden.
   Die erfindungsgemäße Verknüpfung zweier oder mehrerer Fettaminalkylendiamin-Einheiten, die schon an einem Teil der Polyglykoletherketten endständig mit Monocarbonsäuren verestert sind, kann vorzugsweise in einer zweiten Reaktionsstufe durch Veresterung mit einer der vorstehend genannten Dicarbonsäuren durchgeführt werden, wobei vorzugsweise wiederum ein Verhältnis von Fettaminalkylendiamin-Oxalkylatteilester zu Dicarbonsäure von 2:1 bis 10:9 gewählt wird. Im Falle, daß das teilveresterte Blockpolymerisat nur noch eine freie Hydroxygruppe besitzt, ist ein Molverhältnis von etwa 2:1 sinnvoll.
   Auch die Teilester dieser verknüpften Fettaminalkylendiamin-Oxalkylateinheiten sind oberflächenaktive Mittel und können im Sinne der Erfindung eingesetzt werden.
   Es ist auch möglich, die Umsetzung der Fettaminalkylendiamin-Oxalkylate mit Monocarbonsäuren und Dicarbonsäuren in einer Reaktionsstufe durchzuführen, wobei dann in der Regel Gemische von mehrkernigen und einkernigen veresterten Fettaminalkylendiamin-Oxalkylaten entstehen.
b2) Im Falle von eingeführten Maleinsäurehalbestergruppen ist es vorteilhaft, diese Halbestergruppen in die entsprechenden Sufobernsteinsäurehalbestergruppen zu überführen. Dies gelingt beispielsweise nach Zugabe von wäßrigen Lösungen von Sulfiten, Pyrosulfiten oder Hydrogensulfiten zu den Verbindungen, die Maleinsäurehalbestergruppen aufweisen. Auf jede Maleinsäurehalbestergruppe werden dabei 1,0 bis 1,5 Mol, bevorzugt 1,0 bis 1,1 Mol, schwefliger Säure in Form von Alkali- oder Erdalkalisulfiten oder -hydrogensulfiten oder -pyrosulfiten eingesetzt. Die zugesetzte Wassermenge beträgt in der Regel etwa 50 bis 85 Gew.-%, bezogen auf die gesamte Lösung oder Mischung und ist abhängig von der Löslichkeit der Sulfobernsteinsäurehalbestersalze und der Viskosität der Lösungen. Die Reaktionstemperatur bei der Umsetzung der genannten Sulfite mit den Maleinsäurehalbesterverbindungen beträgt in der Regel 20 bis 100°C, bevorzugt 40 bis 80°C.
b3) Zur Einführung von SO₃M-Gruppen werden die nach b) erhältlichen Oxalkylate mit 1 bis 9 Mol, vorzugsweise 1 bis 4 Mol, eines Sulfatierungsmittels, beispielsweise Schwefelsäure, Amidosulfonsäure oder Chlorsulfonsäure oder entsprechenden Anhydriden, pro Mol Oxalkylat umgesetzt. Während bei der Sulfatierung mit Amidosulfonsäure die Ammoniumsalze der Schwefelsäurehalbester gebildet werden, entstehen bei der Ausführungsform mit gasförmigem Schwefeltrioxid in Mischungen mit Inertgas sowie auch bei der Sulfatierung mit Chlorsulfonsäure Schwefelsäurehalbester in der Säureform, aus denen nach c) durch Neutralisation mit entsprechenden anorganischen oder organischen Basen Salze hergestellt werden können. Zu dieser Neutralisation werden bevorzugt die Alkalihydroxide eingesetzt, die zu den sehr gut wasserlöslichen Alkalisalzen der erfindungsgemäßen Schwefelsäurehalbester führen.
   Eine vorteilhafte Ausführungsform besteht in der Bereitstellung von Mischestern aus Harzsäuren, Fettsäuren oder aromatischen Carbonsäuren einerseits und aus den dem Rest Z⁶ zugrundeliegenden Dicarbonsäuren andererseits. Die Herstellung dieser Mischester kann, wie vorstehend erwähnt, durch mindestens zwei hintereinander ablaufende Teilveresterungsschritte erfolgen, wobei zweckmäßigerweise die Veresterung mit der Dicarbonsäure(derivat) der letzte Teilveresterungsschritt ist.
c) Die 1 bis 20, vorzugsweise 2 bis 10, Carboxygruppen oder Sulfogruppen enthaltenden Oxalkylierungsprodukte werden in einer bevorzugten Ausführungsform mit Alkalihydroxiden oder Erdalkalihydroxiden oder bevorzugt mit Basen wie Ammoniak, C₁-C₅-Alkylaminen, C₂-C₅-Alkylolaminen oder deren Alkylenoxidaddukten, wobei pro Mol Amin oder Alkylolamin bis zu 150 Mol Ethylenoxid oder Propylenoxid oder eine Kombination aus beiden angelagert sind oder mit Aminen der Formeln (II) oder (III), neutralisiert.

Als Alkylamine und Alkylolamine kommen beispielsweise in Betracht: Ethylamin, Propylamin, Isopropylamin, n-Butylamin, Isobutylamin, Monoethanolamin, Monopropanolamin, Monoisopropanolamin, Monobutanolamin, Monoisobutanolamin, Diethanolamin, Dipropanolamin, Dibutanolamin, Triethanolamin, Tripropanolamin oder Tributanolamin sowie Di- und Polyamine, wie 1,2-Diaminoethan, 1,3-Diaminopropan, 1,2-Diaminopropan, 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,12-Diaminododecan, Diethylentriamin, Dipropylentriamin, Triethylentetramin, Dipropylentetramin, Tetraethylenpentamin, Tetrapropylenpentamin, Pentaethylenhexamin, Pentapropylenhexamin, Hexaethylenheptamin, Hexapropylenheptamin, Heptaethylenoctamin, Heptapropylenoctamin, 1,3-Diamino-2,2-dimethyl-propan, 1,2-Diamino-2-methyl-propan, 1,3-Diamino-2-methyl-propan, 2,5-Diamino-2,5-dimethylhexan, N-(2-amino-ethyl)-1,3-propylendiamin und N,N'-bis-(3-aminopropyl)ethylendiamin.

Bei der Herstellung der erfindungsgemäßen Verbindungen wird, bedingt durch die Vielzahl der Reaktionszentren in den Ausgangsverbindungen, kein reines, einheitliches Endprodukt erhalten, sondern eine Mischung, die jedoch Verbindungen gemäß Formel (I) als Hauptkomponente` das heißt, zu mehr als 50 Gew.-%, vorzugsweise zu mehr als 90 Gew.-%, enthält.

Nach dem erfindungsgemäßen Verfahren werden insbesondere dann Gemische erhalten, wenn ein nach Reaktionsschritt a) erhältliches Oxalkylat in einer einzigen Reaktionsstufe mit mehreren verschiedenen Carbonsäuren verestert wird. Sind bei dieser Veresterung auch Dicarbonsäuren zugegen, so entstehen in der Regel Gemische von verbrückten und nichtverbrückten Oxalkylatestern.

Die Molmasse der erfindungsgemäßen Verbindungen kann in einem weiten Bereich schwanken und reicht von 1000 bis 20 000, bevorzugt 2 000 bis 10 000, insbesondere 1000 bis 5000.

Die erfindungsgemäßen Verbindungen sind gelbliche oder bräunliche, schaumarme grenzflächenaktive Substanzen, die überraschenderweise für die Herstellung gut fließfähiger und dispersionsstabiler Feststoffdispersionen, insbesondere gut fließfähiger und flockungsstabiler Pigmentpräparationen, geeignet sind.

Die erfindungsgemäßen Verbindungen weisen vielseitige vorteilhatte Eigenschaften auf. Sie gehören zu der Klasse der oberflächenaktiven Verbindungen nach DIN 53900, senken die Oberflächenspannung nach der Ringabreißmethode (DIN 53914) und sind nach den Resultaten im modifizierten Ross-Miles-Test (DIN 53902) als nichtschäumende oder schwachschäumende oberflächenaktive Stoffe zu bezeichnen. Bei geeignetem Hydrophilierungsgrad zeigen sie ausgezeichnetes Netzvermögen für Baumwolle nach der Tauchnetzmethode (DIN 53901) bei gleichzeitig gutem Egalisierverhalten nach DIN 53504. Sie besitzen ein sehr gutes Flockungsschutzvermögen gegenüber Pigmenten und Farbstoffen (DIN 53908) und eine sehr gute Wasserverteilungswirkung als Reinigungsverstärker (DIN 53980) sowie eine gute Auswaschbarkeit als Schmälzmittel (DIN 53504). Die erfindungsgemäßen Verbindungen sind biologisch abbaubar und daher besonders umweltfreundlich.

Die erfindungsgemäßen Verbindungen können aufgrund ihrer vielseitigen oberflächenaktiven Eigenschaften für ein breites Spektrum von Anwendungen eingesetzt werden.

Gegenstand der Erfindung ist deshalb auch die Verwendung einer Verbindung der Formel (I) als oberflächenaktives Mittel. Von besonderem Interesse ist die Verwendung als Kupplungshilfsmittel und Präparationsmittel für die Herstellung von Azofarbmitteln, insbesondere von Azopigmenten, bevorzugt für die Herstellung von Pigmentpräparationen für den wäßrigen Druckfarbensektor; als Dispergier- und Verteilungsmittel für die Feinverteilung und Stabilisierung von Feststoffen, vorzugsweise schwerlöslichen oder unlöslichen, anorganischen und organischen Farbmitteln, vorzugsweise für die Herstellung von gut fließfähigen Pigmentdispersionen für wäßrige Dispersionsanstrichfarben; zum Ein- und Durchfärben- oder Pigmentieren von Leder; sowie zur Formulierung von Dispersionsfarbstoffen, wie sie bevorzugt zum Färben von natürlichen und synthetischen Fasermaterialien wie Baumwolle, Wolle, Zellulose, Zellwolle, Zelluloseacetat, Zellulosetriacetat, Polyester, Polyamid und Polyacrylnitril oder von Fasermaterialien, die diese Stoffe enthalten, verwendet werden.

Die erfindungsgemäßen Verbindungen eignen sich außerdem für den Einsatz als Additive und Emulgatoren, beispielsweise als Korrosionsschutzzusätze und als Zusätze für die Herstellung von Kühlschmiermitteln und Kaltwalzölen in der Metallverarbeitungsindustrie. Weiterhin können die Substanzen als Dispergier- und Emulgiermittel für die Herstellung von Reinigungsverstärkern, Carrier-Emulsionen und Formulierungen für Pflanzenschutz- und Schädlingsbekämpfungsmittel sowie als Zusatzmittel zu Spinnlösungen und für Spinnbäder verwendet werden. Sie eignen sich ferner als Netz-, Egalisier-, Flotations-, Viskoseveredlungs- und Färbereihilfsmittel.

Die erfindungsgemäßen Verbindungen können einzeln oder als Gemische sowie in Kombination mit anderen, nichtionogenen sowie gegebenenfalls mit anionaktiven oder kationaktiven Tensiden oder Gemischen davon eingesetzt werden. Weiterhin können Sie zusammen mit üblichen Mengen an Gerüstsubstanzen oder anderen üblichen Zusätzen oder Hilfsstoffen in Emulgier- und Dispergiermittelformulierungen zur-Anwendung kommen.

In den folgenden Beispielen beziehen sich "Teile" und Prozentangaben auf das Gewicht, Volumenteile verhalten sich zu Gewichtsteilen wie Liter zu Kilogramm. Der Umsetzungsgrad in den vorstehend beschriebenen Reaktionsstufen wird in den Herstellungsbeispielen durch die Bestimmung der Hydroxylzahl, Säurezahl und Aminzahl charakterisiert. Die Säurezahl (SZ) wird nach DIN 53 402 bestimmt. Die Säurezahl gibt die Menge an Kaliumhydroxid in Milligramm an, die zur Neutralisation von 1 g des Reaktionsprodukts verbraucht wird. Die Hydroxylzahl wird nach DIN 53 240 bestimmt und ist ein Maß für den Gehalt an freien Hydroxylgruppen im Molekül; sie entspricht der Menge an Kaliumhydroxid in mg, welche notwendig ist, um die Menge Essigsäure zu neutralisieren, die bei der Acetylierung von 1 g der Prüfsubstanz verbraucht wird. Die Aminzahl wird nach DIN 53 176 bestimmt und ist diejenige Menge an Kaliumhydroxid in Milligramm, die dem Aminanteil von 1 g Substanz äquivalent ist.

### Herstellungsbeispiel 1

### a) N-Talgfettamindipropylendiamin-oxalkylat

200 Teile N-Talgfettamindipropylendiamin wurden unter Rühren und Zuführen von 187 Teilen Propylenoxid ohne Zugabe eines Katalysators unter Aufrechterhaltung eines Druckes von 2 bis 4 bar und einer Temperatur von 130 bis 140°C oxpropyliert. Nach Zugabe von 3,3 Teilen Natriummethylat, 30 %ig in Methanol, und Entfernen des Methanols unter reduziertem Druck wurden 474 Teile Ethylenoxid bei 120 bis 140°C und einem Druck von 3 bis 5 bar zugeführt. Nachdem das gesamte Ethylenoxid aufgedrückt war, wurde 1 Stunde bei 140 bis 150°C gerührt. Das erhaltene zähflüssige, gelbbraune Oxalkylat enthält 6 Propylenoxid-Einheiten und 20 Ethylenoxid-Einheiten und hat eine Hydroxylzahl von 70.

### b) N-Talgfettamindipropylendiamin-oxalkylat-di-kolophoniumester

300 Teile Oxalkylat nach Herstellungsbeispiel 1a) wurden entsprechend der halben Hydroxylzahl mit 56,2 Teilen disproportioniertem Kolophonium auf 70 bis 80°C erwärmt und unter Stickstoffgas eine Stunde lang gerührt. Nach Zugabe von 6 Teilen Zinnpulver, 2,0 Teilen p-Toluolsulfonsäure und 150 Vol.-Teilen Xylol wurde 16 Stunden lang auf 150 bis 160°C erhitzt und das Reaktionswasser durch Auskreisen entfernt. Nach Abdestillieren des Xylols erhielt man ein Produkt mit einer Säurezahl von weniger als 20.

### Herstellungsbeispiel 2

### N-Talgfettamindipropylendiamin-oxalkylat-di-kolophoniumester-dimaleinsäurehalbester-diethylentriaminsalz

300 Teile Kolophoniumteilester nach Herstellungsbeispiel 1b) wurden entsprechend einer restlichen Hydroxylzahl von 35 mit 18,4 Teilen Maleinsäureanhydrid in Gegenwart von 21 Teilen pulverisiertem Ätznatron innerhalb von 4 Stunden bei 75 bis 80°C halbverestert und nach erhaltener Säurezahl von 46,6 mit 28,7 Teilen Diethylentriamin bei 60 bis 70°C neutralisiert. Das erhaltene Produkt ist wasserlöslich, besitzt in Wasser einen pH-Wert von 8,9 bis 9,2 und eine Aminzahl von 193.

### Herstellungsbeispiel 3

### 3a) N-Talgfettamin-dipropylendiamin-oxalkylat-malonat

200 Teile N-Talgfettamindipropylendiamin wurden, wie in Herstellungsbeispiel 1a) beschrieben, mit 126,8 Teilen Propylenoxid umgesetzt und nach Zugabe von 3,3 Teilen Natriummethylat, 30 %ig in Methanol, nach Entfernung des Methanols unter reduziertem Druck mit 572 Teilen Ethlyenoxid oxethyliert. Das erhaltene zähflüssige gelbbraune Oxalkylat enthäft 4 Propylenoxid- und 24 Ethylenoxid-Einheiten bei einer Hydroxylzahl von 136.

Anschließend wurden 600 Teile dieses Oxalkylates mit 25 Teilen Malonsäure bei 70 bis 80°C unter Stickstoffgas erwärmt. Nach Zugabe von 3 Teilen p-Toluolsulfonsäure und 200 Volumenteilen Xylol wurde 10 Stunden lang auf 155 bis 165°C erhitzt und das Reaktionswasser durch Auskreisen entfernt. Nach Abdestillieren des Xylols hat das Produkt eine Säurezahl von weniger als 10, eine Hydroxylzahl von 94,6 und enthält pro Molekül 12 Propylenoxid-Einheiten und 72 Ethylenoxid-Einheiten.

### 3b) N-Talgfettamin-dipropylendiamin-oxalkylat-malonat-trikolophoniumester

300 Teile Oxalkylat nach Herstellungsbeispiel 3a) wurden entsprechend 3/8 der Hydroxylzahl mit 57,3 Teilen disproportioniertem Kolophonium auf 70 bis 80°C erwärmt und unter Stickstoffgas eine Stunde lang gerührt. Nach Zugabe von 6 Teilen Zinnpulver, 2,0 Teilen p-Toluolsulfonsäure und 150 Volumenteilen Xylol wurde 16 Stunden lang auf 150 bis 160°C erhitzt und das Reaktionswasser durch Auskreisen entfernt. Nach Abdestillieren des Xylols erhielt man ein Produkt mit einer Säurezahl von weniger als 15.

### Herstellungsbeispiel 4

### N-Talgfettamin-dipropylendiamin-oxalkylat-malonat-trikolophoniumester-pentasulfosuccinat

300 Teile Kolophoniumteilester nach Herstellungsbeispiel 3b) wurden entsprechend einer Hydroxylzahl von 59 unter Stickstoffgas mit 95 Teilen Maleinsäureanhydrid bei 75 bis 85°C halbverestert und anschließend mit einer Lösung aus 122 Teilen Natriumsulfit in 960 Teilen Wasser bei 75 bis 80°C in 1 bis 3 Stunden umgesetzt. Nach Klarwerden des Ansatzes wurde noch 1 Stunde gerührt. Das erhaltene Hauptprodukt ist ein Sulfobernsteinsäurehalbester mit einer Resthydroxylzahl von 6,3, bei dem alle 5 Polyoxalkylenketten endständig umgesetzt sind.

### Herstellungsbeispiel 5

### 5a) 2-fach N-Palmkernfettamindipropylendiamin-oxalkylat-phthalsäureester

200 Teile N-Palmkernfettamindipropylendiamin wurden, wie in Herstellungsbeispiel 1a) beschrieben, mit 126,5 Teilen Propylenoxid umgesetzt und nach Zugabe von 3,3 Teilen Natriummethylat, 30 %ig in Methanol, nach Entfernung des Methanols unter reduziertem Druck mit 761 Teilen Ethylenoxid oxethyliert. Das erhaltene zähflüssige, gelbbraune Oxalkylat enthält im Mittel 4 Propylenoxid- und 32 Ethylenoxid-Einheiten pro Molekül bei einer Hydroxylzahl von 112.

Anschließend wurden 300 Teile dieses Oxalkylats entsprechend einem Viertel der Hydroxylzahl nach Zugabe von 0,1 Teilen pulverisiertem Ätznatron mit 22,1 Teilen Phthalsäureanhydrid unter Stickstoffgas innerhalb von 4 Stunden bei 110 bis 120°C zum Phthalsäurehalbester umgesetzt. Die gefundene Säurezahl liegt bei 26.

Anschließend wurden weitere 300 Teile des N-Palmkernfettamindipropylendiamin-oxalkylats sowie 1,5 Teile p-Toluolsulfonsäure und 200 Volumenteile Xylol zugegeben und 10 Stunden bei 155 bis 165°C unter Auskreisen des Reaktionswassers verestert. Nach Abdestillieren des Xylols erhielt man ein Produkt mit einer Säurezahl von 12 und einer Hydroxylzahl von 80 bis 82.

### 5b) 2-fach N-Palmkernamindipropylendiamin-oxalkylat-phthalsäureester-trikolophonium-ester

300 Teile Oxalkylat nach Herstellungsbeispiel 5a) wurden entsprechend der halben Hydroxylzahl mit 65,4 Teilen disproportioniertem Kolophonium auf 70 bis 80°C erwärmt und unter Stickstoffgas eine Stunde lang gerührt. Nach Zugabe von 12 Teilen Zinnpulver, 4,0 Teilen p-Toluolsulfonsäure und 150 Volumenteilen Xylol wurde 16 Stunden lang auf 150 bis 160°C erhitzt und das Reaktionswasser durch Auskreisen entfernt. Nach Abdestillieren des Xylols erhielt man ein Produkt mit einer Säurezahl von weniger als 18.

### Herstellungsbeispiel 6

### N-Palmkernfettamindipropylendiamin-oxalkylat-kolophoniumester-1,3-propandiaminsalz

300 Teile Kolophoniumester nach Herstellungsbeispiel 5b) wurden entsprechend einer Resthydroxylzahl von 40,5 mit 21,7 Teilen Bernsteinsäureanhydrid unter Stickstoffstrom bei 100 bis 110°C in 4 Stunden bis zu einer Säurezahl von 35,5 zum Halbester umgesetzt und, auf Säurezahl bezogen, mit 46,8 Teilen 1,3-Propandiamin neutralisiert. Das erhaltene Aminsalz hat einen pH-Wert von 8 bis 8,2 und eine Aminzahl von 82.

### Herstellungsbeispiel 7

### 7a) 2-fach N-Cocosfettamindipropylendiamin-oxalkylat-bernsteinsäureester

200 Teile N-Cocosfettamindipropylendiamin wurden, wie in Herstellungsbeispiel 1a) beschrieben, mit 310 Teilen Propylenoxid umgesetzt und nach Zugabe von 3,3 Teilen Natriummethylat, 30 %ig in Methanol, nach Entfernen des Methanols unter reduziertem Druck mit 571 Teilen Ethylenoxid oxethyliert. Das erhaltene zähflüssige, gelbbraune Oxalkylat enthält im Mittel 8 Propylenoxid- und 44 Ethylenoxid-Einheiten pro Molekül bei einer Hydroxylzahl von 87. Von dem erhaltenen Oxalkylat wurden 300 Teile entsprechend einem 1/4 der Hydroxylzahl mit 47,0 Teilen Bernsteinsäureanhydrid innerhalb von 4 Stunden bei 100 bis 110°C bis zu einer Säurezahl von 65,7 halbverestert und nach Zugabe von 1,5 Teilen p-Toluolsulfonsäure und 322 Teilen Oxalkylat sowie 150 Volumenteilen Xylol in 10 Stunden bei 155 bis 165°C unter Auskreisen des Reaktionswassers verestert. Nach Abdestillieren des Reaktionswassers erhielt man ein über die -OC-CH₂-CH₂-CO-Gruppe verknüpftes zweifaches N-Cocosfettamindipropylendiamin-oxalkylat mit einer Säurezahl unter 10 und einer Hydroxylzahl von 64.

### 7b) 2-fach N-Cocosfettamindipropylendiamin-oxalkylat-bernsteinsäureester-hexa-kolophoniumester

300 Teile Oxalkylat nach Herstellungsbeispiel 7a) wurden entsprechend Hydroxylzahl mit 104 Teilen disproportioniertem Kolophonium auf 70 bis 80°C erwärmt und unter Stickstoffgas eine Stunde lang gerührt. Nach Zugabe von 12 Teilen Zinnpulver, 4,0 Teilen p-Toluolsulfonsäure und 150 Volumenteilen Xylol wurde 16 Stunden lang auf 150 bis 160°C erhitzt und das Reaktionswasser durch Auskreisen entfernt. Nach Abdestillieren des Xylols erhielt man ein Produkt mit einer Säurezahl von weniger als 20.

### Herstellungsbeispiel 8

### 8a) N-Talgfettamindipropylendiamin-oxalkylat

200 Teile N-Talgfettamindipropylendiamin wurden, wie in Herstellungsbeispiel 1a) beschrieben, mit 250,1 Teilen Propylenoxid umgesetzt und nach Zugabe von 3,3 Teilen Natriummethylat, 30 %ig in Methanol, nach Entfernung des Methanols unter reduziertem Druck mit 1364 Teilen Ethylenoxid oxethyliert. Das erhaltene zähflüssige gelbbraune Oxalkylat enthält im Mittel 8 Propylenoxid- und 54,7 Ethylenoxid-Einheiten pro Molekül bei einer Hydroxylzahl von 69,4.

### 8b) N-Talgfettamindipropylendiamin-oxalkylat-bernsteinsäurehalbester

500 Teile Oxalkylat nach Herstellungsbeispiel 8a) wurden entsprechend 1/4 der Hydroxylzahl mit 15,5 Teilen Bernsteinsäureanhydrid auf 100 bis 110°C unter Stickstoff erwärmt und gut gemischt. Nach Zugabe von 0,1 Teilen Ätznatron wurde im gleichen Temperaturbereich 4 Stunden gerührt. Man erhielt ein Produkt mit einer Säurezahl von 16,7.

### 8c) 3-fach-N-Talgfettamindipropylendiamin-oxalkylat-di-bernsteinsäureester

200 Teile Bernsteinsäurehalbester aus Herstellungsbeispiel 8b) wurden entsprechend der Säurezahl mit 193,3 Teilen Oxalkylat nach Herstellungsbeispiel 8a) bei 60 bis 70°C unter Stickstoffgas gut gemischt und nach Zugabe von 3 Teilen p-Toluolsulfonsäure und 150 Volumenteilen Xylol wurde 8 Stunden bei 155 bis 165°C verestert, wobei das Reaktionswasser durch Auskreisen am Wasserabscheider entfernt wurde. Die Säurezahl des erhaltenen Produktes beträgt 8,9.

Anschließend wurden weitere 208,6 Teile Oxalkylat-bernsteinsäurehalbester nach Herstellungsbeispiel 8b) zugegeben und weitere 8 Stunden unter Entfernung des Reaktionswassers bei 155 bis 165°C verestert. Danach wurde das Xylol abdestilliert und die Säurezahl bestimmt. Das Produkt hat eine Säurezahl von weniger als 5 und eine Hydroxylzahl von 40 bis 50. In dem erhaltenen Produkt sind 3 N-Talgfettaminpropylendiamin-oxalkylat-Einheten gemäß Herstellungsbeispiel 8a) durch 2 Bernsteinsäure-Einheiten verknüpft.

### Herstellungsbeispiel 9

### 3-fach N-Talgfettamindipropylendiamin-oxalkylat-bernsteinsäureester-benzoatsulfosuccinat

300 Teile 3-fach-Oxalkylat nach Herstellungsbeispiel 8c) wurden entsprechend der halben Hydroxylzahl mit 14,7 Teilen Benzoesäure nach Zugabe von 2 Teilen Zinnpulver und 0,5 Teilen p-Toluolsulfonsäure sowie 150 Volumenteilen Xylol bei 155 bis 165°C in 12 Stunden bei gleichzeitigem Auskreisen des Reaktionswassers verestert. Nach Abdestillieren des Xylols hatte der vorliegende Benzoesäureteilester eine Säurezahl von weniger als 20.

Anschließend wurde in Gegenwart von 0,1 Teilen pulverisiertem Ätznatron und 11,8 Teilen Maleinsäureanhydrid innerhalb von 3 bis 4 Stunden bei 70 bis 80°C weiterverestert und anschließend durch Zugabe einer Lösung von 15,2 Teilen Natriumsulfit und 634,6 Teilen Wasser in 1 bis 3 Stunden bei 75 bis 80°C zum Sulfosuccinat umgesetzt. Das erhaltene Produkt ist ein Sulfobernsteinsäurehalbester mit einer Resthydroxylzahl von etwa 5,9, bei dem alle 8 Polyoxyalkylengruppen endständig umgesetzt sind.

### Herstellungsbeispiel 10

### 10a) N-Talgfettamindipropylendiamin-oxalkylat

200 Teile N-Talgfettamindipropylendiamin wurden unter Rühren und Zuführen von 190 Teilen Propylenoxid und 1443,8 Teilen Ethylenoxid analog Herstellungsbeispiel 1a) oxalkyliert. Das erhaltene wachsweiche Produkt enthält 6 Propylenoxid- und 50 Ethylenoxid-Einheiten pro Molekül und besitzt eine Hydroxylzahl von 76,7.

### 10b) N-Talgfettamindipropylendiamin-oxalkylat-tetra-ölsäureester

300 Teile Oxalkylat nach Herstellungsbeispiel 10a) wurden entsprechend Hydroxylzahl nach Zugabe von 1,5 Teilen p-Toluolsulfonsäure und 150 Volumenteilen Xylol mit 115,7 Teilen handelsüblicher Ölsäure bei 155 bis 165°C innerhalb von 12 Stunden unter Auskreisen des Reaktionswassers verestert. Nach Abdestillieren des Xylols erhielt man ein Produkt mit einer Säurezahl von weniger als 20, bei dem alle 4 Polyoxyalkylengruppen endständig verestert sind.

### Herstellungsbeispiel 11

### 11a) N-Talgfettamindipropylendiamin-oxalkylat-maleinsäurehalbester

500 Teile Oxalkylat nach Herstellungsbeispiel 8a) wurden entsprechend 1/4 der Hydroxylzahl mit 15,1 Teilen Maleinsäureanhydrid auf 70 bis 80°C unter Stickstoff erwärmt und gut gemischt. Nach Zugabe von 0,1 Teilen Ätznatron wurde im gleichen Temperaturbereich 4 Stunden gerührt. Man erhielt ein Produkt mit einer Säurezahl von 16,4.

### 11b) 3-fach-N-Talgfettamindipropylendiamin-oxalkylat-maleinsäureester

200 Teile Maleinsäurehalbester nach Herstellungsbeispiel 11a) wurden entsprechend der Säurezahl mit 189,8 Teilen Oxalkylat nach Herstellungsbeispiel 8a) bei 60 bis 70°C in Stickstoffatmosphäre gut gemischt. Nach Zugabe von 3 Teilen p-Toluolsulfonsäure und 150 Volumenteilen Xylol wurde 8 Stunden bei 155 bis 165°C verestert, wobei das Reaktionswasser durch Auskreisen am Wasserabscheider entfernt wurde. Man erhielt ein Produkt mit einer Säurezahl von 8,5.

Anschließend wurden weitere 197,5 Teile Oxalkylat-maleinsäurehalbester nach Herstellungsbeispiel 11a) zugegeben und weitere 8 Stunden unter Entfernung des Reaktionswassers bei 155 bis 165°C verestert. Danach wurde das Xylol abdestilliert und die Säurezahl bestimmt. Das Produkt hat eine Säurezahl von weniger als 5 und eine Hydroxylzahl von 40 bis 50. In dem erhaltenen Produkt sind 3 N-Talgfettamindipropylendiamin-oxalkylat-Einheiten durch 2 Maleinsäure-Einheiten verknüpft.

### 11c) 3-fach N-Talgfettamindipropylendiamin-oxalkylat-dimaleinsäureester-phthalsäureester-1,3-propandiamin-salz

200 Teile 3-fach-Oxalkylat nach Herstellungsbeispiel 11b) wurden auf etwa 50°C erwärmt und portionsweise 23,7 Teile Phthalsäure entsprechend vorliegender Hydroxylzahl eingetragen und 3 bis 4 Stunden bei 100 bis 120°C unter Stickstoff gerührt. Man erhielt einen gelbbraunen, zähflüssigen Phthalsäurehalbester mit einer Säurezahl von 44,3. Nach Abkühlung auf 55 bis 65°C wurden entsprechend der Säurezahl durch Eintragen von 41,6 Teilen 1,3-Propandiamin und Erhöhung der Reaktionstemperatur auf 65 bis 75°C in etwa 1 Stunde zum Aminsalz umgesetzt. Man erhielt ein gelbbraunes Aminprodukt mit einer Aminzahl zwischen 80 und 90.

### Herstellungsbeispiel 12

### N-Talgfettamindipropylendiamin-oxalkylat-di-ölsäureester-disulfat

300 Teile N-Talgfettamindipropylendiamin-oxethylat nach Herstellungsbeispiel 10a) wurden gemäß halber Hydroxylzahl nach Zugabe von 1,2 Teilen p-Toluolsulfonsäure und 150 Volumenteilen Xylol mit 57,8 Teilen handelsüblicher Ölsäure bei 155 bis 165°C in 10 Stunden und Entfernung des Reaktionswassers verestert. Nach Abdestillieren des Xylols wurde noch 2 Stunden bei 170 bis 180°C bis zu einer Säurezahl unter 18 nachverestert.

Anschließend wurde mit 250 Volumenteilen Methylenchlorid verdünnt und entsprechend Resthydroxylzahl 47,8 Teile Chlorsulfonsäure bei 15 bis 20°C zugetropft, wobei man trockenen Stickstoff in schwachem Strom durch die Lösung leitete, der das entweichende Chlorwasserstoffgas über einen Rückflußkühler entfernte. Gegen Ende der Reaktion wurde auf 30°C erwärmt und gerührt, bis kein Chlorwasserstoffgas mehr entwich. Nach Abdestillieren des Methylenchlorids unter vermindertem Druck bei 30°C blieben 352 g eines Öles mit einem Gehalt von 0,04 % titrierbarem Chlor im Rückstand. Zur Neutralisation wurden 900 Teile Wasser zugegeben und mit 34,5 Teilen Natronlauge (33 %ig) bis pH 7,0 neutralisiert. Das erhaltene Hauptprodukt ist ein Schwefelsäurehalbester mit einer Resthydroxylzahl von 8,1, bei dem alle 4 Polyoxalkylenketten endständig umgesetzt sind.

### Herstellungsbeispiel 13

### 13a) N-Talgfettamindipropylendiamin-oxalkylat

200 Teile N-Talgfettamindipropylendiamin wurden unter Rühren und Zuführen von 253 Teilen Propylenoxid und 1443,8 Teilen Ethylenoxid analog Herstellungsbeispiel 1a) oxalkyliert. Das erhaltene wachsweiche Produkt enthält 8 Propylenoxid- und 50 Ethylenoxid-Einheiten pro Molekül und besitzt eine Hydroxylzahl von 70,0.

### 13b) 2-fach N-Talgfettamindipropylendiamin-oxalkylatdimer-fettsäureester

371 Teile Oxalkylat nach Herstellungsbeispiel 13a) wurden mit 282 Teilen handelsüblicher Dimerfettsäure (®Pripol 1022) nach Zugabe von 2 Teilen p-Toluolsulfonsäure und 150 Volumenteilen Xylol innerhalb von 8 Stunden bei 155 bis 165°C verestert, wobei das Reaktionswasser durch Auskreisen am Wasserabscheider entfernt wurde. Danach wurde das Xylol abdestilliert und man erreichte eine Säurezahl von 6,0. Das erhaltene Produkt enthält 2 Oxalkylat-Einheiten, die durch eine Dimerfettsäure-Einheit verknüpft ist.

### Herstellungsbeispiel 14

### 14a) N-Talgfettamindimethyldipropylendiamin-oxalkylat

200 Teile N-Talgfettamindimethyldipropylendiamin wurden unter Rühren und Zuführen von 249 Teilen Propylenoxid ohne Zugabe eines Katalysators unter Aufrechterhalten eines Druckes von 2 bis 4 bar bei 130°C bis 140°C oxpropyliert. Nach Zugabe von 3,3 Teilen Natriummethylat, 30 %ig in Methanol, und Entfernen des Methanols unter reduziertem Druck, wurden 474 Teile Ethylenoxid bei 120 bis 140°C bei 3 bis 5 bar zugeführt. Nachdem das gesamte Ethylenoxid aufgedrückt war, wurde 1 Stunde bei 140 bis 150°C gerührt. Das erhaltene zähflüssige, gelbbraune Oxalkylat enthält 8 Propylenoxid- und 20 Ethylenoxid-Einheiten pro Molekül und besitzt eine Hydroxylzahl von 65.

### 14b) N-Talgfettamindimethyldipropylendiamin-oxalkylat-di-kolophoniumester

300 Teile Oxalkylat nach Herstellungsbeispiel 14a) wurden entsprechend der halben Hydroxylzahl mit 52,5 Teilen disproportioniertem Kolophonium auf 70 bis 80°C erwärmt und unter Stickstoffgas eine Stunde lang gerührt. Nach Zugabe von 6 Teilen Zinnpulver, 2,0 Teilen p-Toluolsulfonsäure und 150 Volumenteilen Xylol wurde 16 Stunden lang auf 150 bis 160°C erhitzt und das Reaktionswasser durch Auskreisen entfernt. Nach Abdestillieren des Xylols erhielt man ein Produkt mit einer Säurezahl von weniger als 18.

### 14c) N-Talgfettamindimethyldipropylendiaminoxethylat-di-kolophoniumester-dimaleinsäurehalbester-diethylentriaminsalz

300 Teile Kolophoniumester nach Herstellungsbeispiel 14b) wurden entsprechend einer restlichen Hydroxylzahl von 32,5 mit 17 Teilen Maleinsäureanhydrid in Gegenwart von 21 Teilen pulverisiertem Ätznatron innerhalb von 4 Stunden bei 75 bis 80°C halbverestert und nach erhaltener Säurezahl von 45,6 mit 18,8 Teilen Diethylentriamin bei 60 bis 70°C neutralisiert. Das erhaltene Produkt ist wasserlöslich und besitzt einen pH-Wert von 8,3 bis 8,5 und eine Aminzahl von etwa 158.

### Anwendungsbeispiel 1

120,4 Teile 1-Acetoacetylamino-2,4-dimethylbenzol und 12,2 Teile 1-Acetoacetylamino-2,5-dimethoxy-4-chlorbenzol wurden in 1800 Teilen Wasser und 62 Volumenteilen 33 %iger Natronlauge gelöst und nach Zusatz von 3,0 Teilen eines Fettalkoholpolyglykolethers mit Hilfe von 22 Volumenteilen Essigsäure gefällt. Nach Zugabe von 5,0 Teilen des Produkts aus Herstellungsbeispiel 2 kuppelte man mit einer Lösung von tetrazotiertem 4,4'-Diamino-3,3'-dichlordiphenyl, wobei die Tetrazoniumsalzlösung durch Zugabe von 120 Volumenteilen wäßriger 5-normaler Natriumnitritlösung zu einem Gemisch aus 76 Teilen 4,4'-Diamino-3,3'-dichlordiphenyl, 366 Volumenteilen 5-normaler Salzsäure und 1040 Teilen Wasser hergestellt wurde. Nach beendeter Kupplung wurde die Pigmentsuspension mit 2,5 Teilen Dehydroabietylamin versetzt, alkalisch gestellt, anschließend mit einer Lösung, die 3,6 Teile des Produkts aus Herstellungsbeispiel 7b) und 36 Teile eines partiell hydrierten Kolophoniums enthielt, versetzt und 30 Minuten bei 98°C erhitzt. Danach stellte man mit Salzsäure pH 4 ein und erhitzte weitere 30 Minuten bei 98°C. Anschließend wurde filtriert, gewaschen und getrocknet. Man erhielt eine Pigmentpräparation, die beim Einarbeiten in einen Druckfarbenfirnis für den Buch- und Offsetdruck eine Druckfarbe mit sehr guten anwendungstechnischen Eigenschaften ergab. Die Druckfarbe zeichnet sich im Vergleich zu einer Druckfarbe, die ohne den Zusatz der Produkte aus den Herstellungsbeispielen 2 und 7b) hergestellt worden ist, durch ein deutlich verbessertes Fließverhalten bei gleichzeitiger Erhöhung der Farbstärke und des Ganzes aus.

### Anwendungsbeispiel 1a)

Die in Anwendungsbeispiel 1 verwendeten Produkte aus den Herstellungsbeispielen 2 und 7b wurden durch die Produkte aus den Herstellungsbeispielen 6 und 11c ersetzt. Man erhielt eine Pigmentpräparation mit ähnlich guten Eigenschaften wie in Anwendungsbeispiel 1 beschrieben.

### Anwendungsbeispiel 2

506 Teile 3,3'-Dichlor-4,4'-diamino-diphenyl wurden mit 3000 Teilen Wasser und 1250 Volumenteilen 30%iger Salzsäure angerührt und anschließend mit 526 Volumenteilen 40 %iger Natriumnitritlösung bei 0 bis 15°C bisdiazotiert. Zur Vorbereitung der Kupplungsreaktion wurden 733 Teile Acetoacetylaminobenzol in 5000 Teilen Wasser und 400 Volumenteilen 33%iger Natronlauge gelöst, mit 80 Teilen des nach Herstellungsbeispiel 7b hergestellten Tensids versetzt und durch Zugabe von 350 Volumenteilen 80%iger Essigsäure gefällt. Die Azokupplung erfolgte unter langsamem Zulaufen der hergestellten Lösung des Bis-diazoniumsalzes zur Suspension der gefällten Kupplungskomponente, wobei der pH-Wert durch laufende Zugabe von 6%iger Natronlauge bei etwa 4,5 gehalten wurde. Nach Beendigung der Kupplung wurde die essigsaure Suspension auf 50°C erwärmt und mit 75 Teilen Dodecylbenzyl-dimethyl-ammoniumchlorid, 375 Teilen Talgfettamin-dipropylendiamin und 150 Teilen Bis-(4-aminocyclohexyl)-methan versetzt. Man erhitzte auf 90 bis 100°C und hielt diese Temperatur eine halbe Stunde lang aufrecht. Dann wurde die Mischung mit 750 Volumenteilen 33%iger Natronlauge alkalisch gestellt und einige Stunden bei 90 bis 100°C gerührt. Anschließend wurde das Produkt filtriert, gewaschen, getrocknet und gemahlen. Man erhielt eine Präparation von C.I. Pigment Yellow 12 (C.I. No. 21090), die sich gut zum Pigmentieren von Tiefdruckfarben auf Toluolbasis verwenden läßt. Die erhaltene Pigmentpräparation von Pigment Yellow 12 zeigt sehr gute Resultate bezüglich Farbstärke, Glanz, Transparenz und Gradationsverhalten. Die damit pigmentierten Tiefdruckfarben zeichnen sich auch durch eine vorteilhafte niedrige Viskosität aus.
Ebenso gute Ergebnisse erhielt man bei Einsatz einer Kombination der erfindungsgemäßen Verbindungen aus den Herstellungsbeispielen 7b und 10b.

### Anwendungsbeispiel 3

1012 Teile 3,3'-Dichlor-4,4'-diamino-diphenyl wurden mit 6000 Teilen Wasser und 2500 Volumenteilen 30%iger Salzsäure angerührt und anschließend mit 1052 Volumenteilen 40 %iger Natriumnitritlösung bei 0 bis 15°C bis-diazotiert. Zur Vorbereitung der Kupplungsreaktion wurden 1396 Teile 1-Acetoacetylamino-2,4-dimethylbenzol und 341 Teile 1-Acetoacetylamino-2-methoxybenzol in 10000 Teilen Wasser und 830 Volumenteilen 33 %iger Natronlauge gelöst. Diese Lösung wurde mit Eis auf 10°C gestellt und mit 40 Volumenteilen einer 10%igen Lösung des Produkts (100 %ig) aus Herstellungsbeispiel 2 versetzt. Danach wurde die Kupplungskomponente mit 1140 Volumenteilen 50 %iger Essigsäure gefällt. Die Kupplung erfolgte durch langsames Zulaufen der vorstehend genannten Bis-diazoniumsalzlösung zur Suspension der gefällten Kupplungskomponente, wobei der pH-Wert mit 6 %iger Natronlauge bei etwa 4,5 gehalten wurde. Nach Beendigung der Kupplung wurde die Pigmentsuspension mit 1100 Volumenteilen 33 %iger Natronlauge und mit einer 80°C heißen Lösung, bestehend aus 750 Teilen eines Balsamharzes, 4000 Volumenteilen Wasser und 220 Volumenteilen 33 %iger Natronlauge, versetzt. Man erhitzte auf 98°C, hielt diese Temperatur für 2 Stunden und gab dann 2000 Volumenteile 1-normaler Salzsäure zu. Anschließend wurde filtriert, gewaschen und getrocknet.

Man erhielt eine Pigmentpräparation, die beim Einfärben in einen Druckfarbenfirnis zum Druck mit verschiedenen Unterlagen vewendet werden kann. Die Druckfarbe zeichnet sich im Vergleich zu einer Druckfarbe, die ohne Zusatz des Produktes aus dem Herstellungsbeispiel 2 hergestellt worden war, durch eine erhöhte Farbstärke, erhöhten Glanz und durch ein deutlich verbessertes Fließverhalten aus.

Gleich gute Ergebnisse erhielt man beim Einsatz der erfindungsgemäßen Verbindung aus dem Herstellungsbeispiel 6.

### Anwendungsbeispiel 4 (Vergleichsbeispiel)

44 Teile Dinitroanilin wurden in üblicher Weise in 91 Teilen Schwefelsäure, 96 %ig, gelöst und in 179,3 Volumenteilen Salzsäure, 34 %ig, bei 0°C eingetragen. Anschließend wurde bei der gleichen Temperatur mit 34,9 Volumenteilen 40%iger wäßriger Natriumnitritlösung diazotiert.

Aus einer Lösung von 34,56 Teilen ß-Naphthol in einer Mischung von 400 Teilen Wasser und 32 Teilen 33%iger Natronlauge wurde reines ß-Naphthol gefällt, indem man die Lösung zu einer Mischung von 1200 Teilen Wasser und 40 Teilen 31 %iger Salzsäure tropfte.

Die Kupplung zum C.I. Pigment Orange 5 (C.I. No. 12075) erfolgte in üblicher Weise durch Zulauf der geklärten Diazoniumsalzlösung zur Suspension des gefällten ß-Naphthols. Anschließend wurde das Pigment filtriert, mit Wasser gewaschen und schließlich getrocknet.

### Anwendungsbeispiel 4a

Man kuppelte C.I. Pigment Orange 5 (C.I. No. 12075), wie in Anwendungsbeispiel 4 beschrieben, wobei man aber vor dem Eintropfen der ß-Naphthol-Lösung noch 4 Teile der erfindungsgemäßen Verbindung aus Herstellungsbeispiel 2 zur vorgelegten Mischung aus 1200 Teilen Wasser und 40 Teilen 31 %iger Salzsäure gab.

Das so erhaltene Pigment unterscheidet sich von dem nach Anwendungsbeispiel 4 hergestellten durch eine deutlich gelbere Nuance und eine wesentlich höhere Farbstärke sowohl im Buch- und Offsetdruck als auch in wäßrigen Präparationen für Flexodruck oder Dispersionsfarben. Die Druckfarben und Präparationen zeichnen sich durch niedrige Viskosität aus. Außerdem zeigt das so erhaltene Pigment im Buch- und Offsetdruck stärkeren Glanz und bessere Transparenz.

### Anwendungsbeispiel 5

138 Teile C.I. Pigment Red 112 (C.I. No. 12370) wurden mit 127 Teilen einer 35%igen wäßrigen Lösung der Verbindung aus Herstellungsbeispiel 9 und 134 Teilen Wasser in einer Rührwerkskugelmühle mit Siliquarzit-Perlen (1 mm Durchmesser) gemahlen und anschließend durch Zugabe von 129 Teilen Wasser verdünnt. Die auf diese Weise erhaltene sehr gut fließfähige Pigmentdispersion eignet sich hervorragend zum Ein- und Durchfärben von Leder, für die Einstellung von Tief- und Flexodruckfarben sowie für die Papiermassefärbung.

### Anwendungsbeispiel 6

138 Teile C.I. Pigment Brown 1 (C.I. Nr. 12480) wurden mit 125 Teilen der erfindungsgemäßen Verbindung aus Herstellungsbeispiel 9 und 135 Teilen Wasser in einer Rührwerkskugelmühle mit Siliquarzit-Perlen (1 mm Durchmesser) gemahlen und anschließend durch Zugabe von 129 Teilen Wasser verdünnt. Die gut fließfähige und lagerstabile Pigmentdispersion eignet sich besonders zum Ein- und Durchfärben von Leder.

### Anwendungsbeispiel 7

138 Teile C.I. Pigment Yellow 83 (C.I. Nr. 21108) wurden entsprechend Anwendungsbeispiel 6 mit 44 Teilen eines nach Herstellungsbeispiel 11b erhältlichen Produktes und mit 217 Teilen Wasser in einer Rührwerkskugelmühle mit Siliquarzit-Perlen (1 mm Durchmesser) gemahlen und aufgearbeitet. Die erhaltene Pigmentdispersion ist sehr gut fließfähig, besitzt eine gute Lagerstabilität und kann sehr gut im Buch- und Offsetdruck oder auch zum Ein- und Durchfärben von Leder eingesetzt werden.

### Anwendungsbeispiel 8

100 Teile C.I. Sulfur Brown 51 (C.I. Nr. 53 327) wurden mit 229 Teilen einer 35 %igen wäßrigen Lösung der Verbindung aus Herstellungsbeispiel 9 entsprechend Anwendungsbeispiel 6 gemahlen und mit 71 Teilen Wasser verdünnt. Die erhaltene Pigmentdispersion eignet sich vorzugsweise zum Ein- und Durchfärben von Leder nach den Auf- und Ausziehverfahren.

### Anwendungsbeispiel 9

50 Teile C.I. Disperse Orange 13 (C.I. Nr. 26 080) wurden mit 85 Teilen eines Produktes gemäß Herstellungsbeispiel 9 und 65 Teilen Wasser in einer Rührwerksperlmühle 4 Stunden bis zur Feinverteilung gemahlen. Nach Zugabe von 50 Teilen Wasser erhielt man einen 20 %igen Farbteig mit sehr guter Feinverteilung, der allen coloristischen Anforderungen, besonders beim Färben von Polyester-, Polyester/Wolle- und Polyester/Zellwolle-Mischgespinsten genügt.

### Anwendungsbeispiel 10

70 Teile eines aromatenhaltigen Mineralöls wurden mit 20 Teilen des Ölsäureesters nach Herstellungsbeispiel 10b und 10 Teilen des Diethylentriaminsalzes nach Herstellungsbeispiel 2 homogen verrührt und anschließend mit Wasser auf 900 Volumenteile verdünnt. Man erhielt eine feindisperse bis transparente Mineralöl-Emulsion, die nach einer Verdünnung von 1:9 neben einer hervorragenden Stabilität über längere Zeit zusätzlich Korrosionsschutzeigenschaften nach DIN 51 360 und als Kühlmittel für beispielsweise spanabhebende Metallbearbeitung eingesetzt werden kann.

### Anwendungsbeispiel 11

70 Teile eines rein aliphatischen Mineralöls wurden mit 20 Teilen eines Produktes gemäß Herstellungsbeispiel 10b und 10 Teilen eines Produktes gemäß Herstellungsbeispiel 3b homogen verrührt und anschließend mit Wasser auf 900 Volumenteile verdünnt. Man erhielt eine feindisperse bis transparente Mineralöl-Emulsion, die nach einer Verdünnung von 1:10 neben einer hervorragenden Stabilität über längere Zeit zusätzlich Korrosionsschutzeigenschaften nach DIN 51 360 besitzt und als Schmälzmittel für die kontinuierliche Kammgarnspinnerei verwendet werden kann.

### Anwendungsbeispiel 12

50 Teile des Pflanzenschutzmittels 2-Carbomethoxy-amino-benzimidazol wurden mit 68 Teilen einer 35%igen wäßrigen Lösung einer Verbindung aus Herstellungsbeispiel 9 und 82 Teilen Wasser bis zur Feinverteilung in einer schnellaufenden 1-Liter-Rührwerksperlmühle gemahlen. Nach Abtrennung der Mahlkörper erhielt man eine sehr stabile Dispersion mit einer guten Schwebefähigkeit ohne Bodensatz. Gleich gute Ergebnisse erhielt man bei Einsatz der erfindungsgemäßen Verbindung aus Herstellungsbeispiel 4.

## Patentansprüche

1. Verbindung der Formel (I) 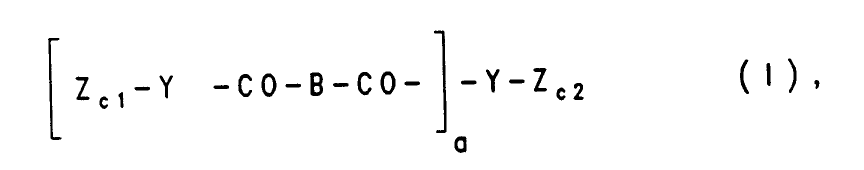 worin
a eine Zahl von 0 bis 9 bedeutet;
c1 und c2 gleich oder verschieden sind und eine Zahl von 4 bis 22 bedeuten;
Y jeweils für gleiche oder verschiedene Einheiten der Formel (Ia) steht 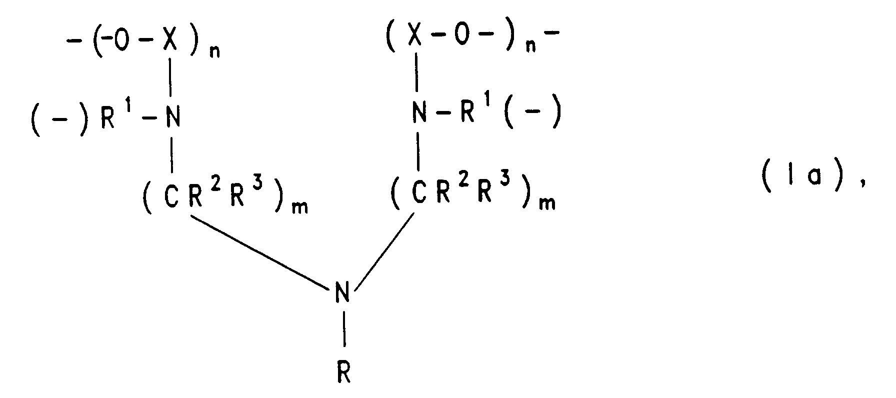
worin
X für eine Gruppe der Formeln -CH₂CH₂-, -CH(CH₃)CH₂- und -CH₂-CH(CH₃)-oder für eine Kombination davon steht;
R für einen gesättigten oder ungesättigten geradkettigen oder verzweigten C₁₂-C₂₂-Alkylrest,
R¹ für ein Wasserstoffatom oder eine divalente Gruppe der Formel -(X-O)ₙ-mit einer wie vorstehend definierten, durch (-) angedeuteten freien Valenz,
R² und R³ gleich oder verschieden sind und ein Wasserstoffatom oder eine Methylgruppe,
m eine Zahl von 2 bis 3 und
n gleiche oder verschiedene Zahlen von 1 bis 200 bedeuten;
Z jeweils für gleiche oder verschiedene Reste Z¹ bis Z⁷ steht, worin
Z¹ Wasserstoff,
Z² einen Acylrest einer geradkettigen, gesättigten oder ungesättigten C₂-C₂₂-Carbonsäure, die unsubstituiert oder durch ein oder zwei Hydroxygruppen substituiert ist,
Z³ einen Acylrest einer Di- oder Tricarbonsäure auf Basis einer di- oder trimerisierten C₈-C₂₄-Fettsäure,
Z⁴ einen Acylrest der Formel R⁴-CO-, in der R⁴ einen Phenyl-, Naphthyl-, Hydroxyphenyl- oder Hydroxynaphthylrest bedeutet,
Z⁵ einen Acylrest einer unmodifizierten oder modifizierten natürlichen Harzsäure,
Z⁶ gleiche oder verschiedene Reste der Formeln -CO-CH=CH-COOM, -CO-(CH₂)_{q}-COOM, -CO-CH₂-CH(SO₃M)-COOM, -CO-CH(SO₃M)-CH₂-COOM, -OC-C₆H₄-COOM, worin q für eine ganze Zahl von 0 bis 10 steht und
Z⁷ -SO₃M bedeuten,
worin M für Wasserstoff; ein Alkalimetall; ein Äquivalent eines Erdalkalimetalls; einen Oxalkylrest der Formel (X-O-)ₙH; eine Ammoniumgruppe, die unsubstituiert oder durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 1 bis 150 Ethylenoxid- oder Propylenoxideinheiten oder einer Kombination von Ethylenoxid- und Propylenoxideinheiten erhaltene Ammoniumgruppe; oder eine Gruppe der Formel (II) 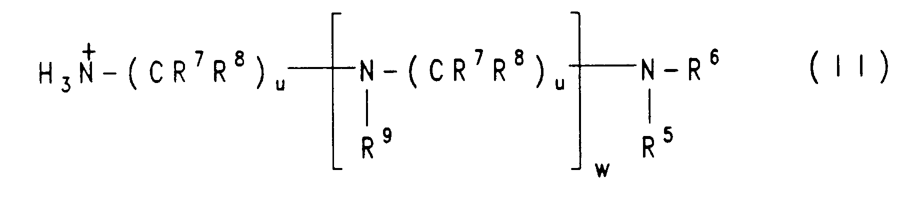 ist, worin R⁹, R⁵ und R⁶ gleich oder verschieden sind und für ein Wasserstoffatom oder eine Hydroxyalkylgruppe mit 1 bis 6 C-Atomen, und R⁷ und R⁸ gleich oder verschieden sind und für Wasserstoff oder Methyl stehen, u jeweils gleich oder verschieden ist und eine ganze Zahl von 2 bis 14 bedeutet und w für eine ganze Zahl von Null bis 25 steht; oder worin M für eine Gruppe der Formel (III) steht 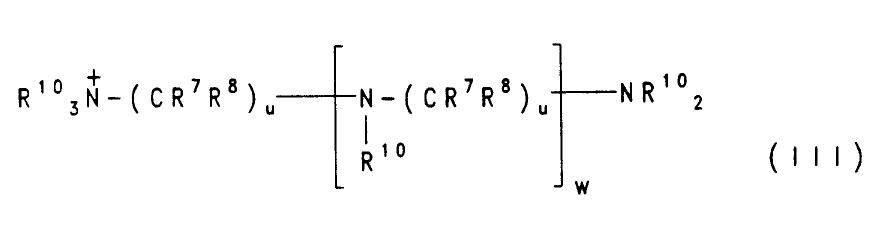 worin R¹⁰ die Gruppe H-(O-X-)_{y} bedeutet, worin X die vorstehend genannten Bedeutungen hat und y eine ganze Zahl von 1 bis 100 ist, und
B für einen geradkettigen, verzweigten oder cyclischen aliphatischen Rest mit jeweils 1 bis 60 C-Atomen, für C₆-C₁₂-Arylen oder für eine Gruppe der Formel -CH=CH-, -CH(SO₃M)CH₂- oder -CH₂CH(SO₃M)- steht, worin M für ein Kation oder für einen Rest der Formel -(X-O-)ₙH steht;
und wobei mindestens ein Rest Z ein Rest aus der Gruppe Z², Z³, Z⁴, Z⁵ und Z⁶ ist; oder wobei mindestens zwei Einheiten der Formel (Ia) über eine divalente Gruppe -CO-B-CO- miteinander verknüpft sind und Z die Bedeutung von Z¹ bis Z⁷ hat.

2. Verbindung nach Anspruch 1, dadurch gekennzeichnet, daß a eine Zahl von 1 bis 4 bedeutet;
und c1 und c2 gleich oder verschieden sind und eine Zahl von 6 bis 12 bedeuten.

3. Verbindung nach Anspruch 1 oder 2, dadurch gekennzeichnet, daß
R für einen gesättigten oder ungesättigten geradkettigen oder verzweigten C₁₂-C₁₈-Alkylrest;
X zu 50 bis 100 %, vorzugsweise zu 80 bis 100 %, aller Reste X für eine Gruppe der Formel -CH₂CH₂- und zu 0 bis 50 %, vorzugsweise zu 0 bis 20 %, aller Reste X für eine Gruppe der Formeln -CH(CH₃)CH₂- oder -CH₂-CH(CH₃)-;
B für C₁-C₆-Alkylen, 1,2-, 1,3- oder 1,4-Phenylen, eine Gruppe der Formeln -CH=CH-, -CH(SO₃M)CH₂- oder -CH₂CH(SO₃M)-, worin M für ein Kation steht,
m für die Zahl 3 und
n für gleiche oder verschiedene Zahlen von 1 bis 50, vorzugsweise 5 bis 30, stehen.

4. Verbindung nach mindestens einem der Ansprüche 1 bis 3, dadurch gekennzeichnet, daß R² und R³ Wasserstoffatome sind.

5. Verbindung nach mindestens einem der Ansprüche 1 bis 4, dadurch gekennzeichnet, daß 20 bis 100 %, vorzugsweise 30 bis 70 %, der Reste Z jeweils unabhängig voneinander die Bedeutung von Z², Z³, Z⁴, Z⁵, Z⁶ oder einer Kombination davon haben und 80 bis 0 %, vorzugsweise 70 bis 30 %, der Reste Z die Bedeutung von Z¹, Z², Z⁴, Z⁵ oder einer Kombination davon haben.

6. Verbindung nach mindestens einem der Ansprüche 1 bis 4, dadurch gekennzeichnet, daß a eine Zahl von 1 bis 4 ist und Z die Bedeutung Z¹ hat.

7. Verbindung nach mindestens einem der Ansprüche 1 bis 5, dadurch gekennzeichnet, daß die dem Acylrest Z² zugrundeliegende Säure eine geradzahlige Fettsäure oder Hydroxyfettsäure mit 8 bis 20, vorzugsweise 12 bis 18, Kohlenstoffatomen ist, insbesondere Tallölfettsäure, Talgfettsäure, Kokosölfettsäure, Palmölfettsäure, Leinölfettsäure, Rizinusölfettsäure oder Rizinensäure.

8. Verbindung nach mindestens einem der Ansprüche 1 bis 5, dadurch gekennzeichnet, daß die dem Acylrest Z⁴ zugrundeliegende Säure Benzoesäure, Salicylsäure, o-, m- oder p-Methylbenzoesäure, Naphthoesäure oder eine Hydroxynaphthoesäure ist.

9. Verbindung nach mindestens einem der Ansprüche 1 bis 5, dadurch gekennzeichnet, daß die dem Acylrest Z⁵ zugrundeliegende Harzsäure eine Harzsäure ist, die in handelsüblichen Kolophoniumarten vorliegt oder eine Kolophoniumart ist.

10. Verbindung nach mindestens einem der Ansprüche 1 bis 5, dadurch gekennzeichnet, daß die dem Acylrest Z⁵ zugrundeliegende Säure Oxalsäure, Malonsäure, Bernsteinsäure, Glutarsäure, Adipinsäure, Pimelinsäure, Korksäure, Acelainsäure, Sebacinsäure, Phthalsäure, Isophthalsäure, Terephthalsäure, Maleinsäure, Fumarsäure oder Sulfobernsteinsäure, vorzugsweise Bernsteinsäure, Phthalsäure, Terephthalsäure, Maleinsäure oder Sulfobernsteinsäure, insbesondere Sulfobernsteinsäure, ist.

11. Verbindung nach mindestens einem der Ansprüche 1 bis 5 und 10, dadurch gekennzeichnet, daß
M für Wasserstoff; ein Alkalimetall; ein Äquivalent eines Erdalkalimetalls; eine Ammoniumgruppe, die durch ein bis vier C₁-C₅-Alkylreste oder ein bis vier C₂-C₅-Alkylolreste substituiert ist; eine aus Ammoniak oder aus C₁-C₅-Alkylaminen oder C₂-C₅-Alkylolaminen durch Anlagerung von 5 bis 30 Ethylenoxid- oder Propylenoxideinheiten oder einer Kombination davon erhaltene Ammoniumgruppe; eine Gruppe der Formel (II) 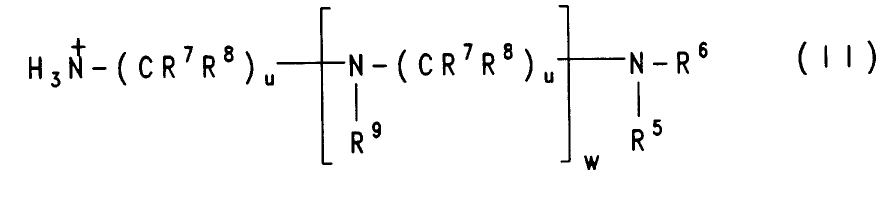 worin R⁹, R⁵ und R⁶ gleich oder verschieden sind und für ein Wasserstoffatom oder Hydroxyalkyl mit 2 bis 3 C-Atomen und R⁷ und R⁸ gleich oder verschieden sind und für Wasserstoff oder Methyl stehen, u jeweils gleich oder verschieden ist und eine ganze Zahl von 2 bis 3 bedeutet und w für eine ganze Zahl von Null bis 5 steht; oder worin M für eine Gruppe der Formel (III) steht 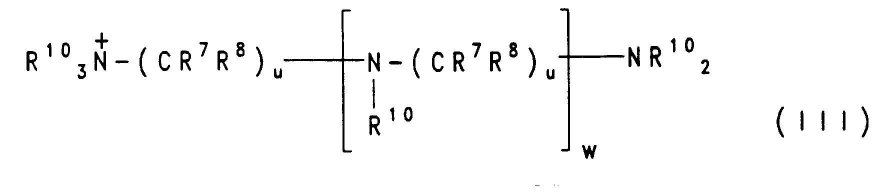 worin R¹⁰ die Gruppe H(-O-X-)_{y} bedeutet,
worin X die vorstehend genannten Bedeutungen hat und y eine ganze Zahl von 1 bis 30 ist.

12. Verfahren zur Herstellung einer Verbindung nach mindestens einem der Ansprüche 1 bis 11, dadurch gekennzeichnet, daß man
a) ein der Formel (Ia) zugrundeliegendes Alkylaminodialkylendiamin mit Ethylenoxid oder Propylenoxid oder beiden Epoxiden hintereinander oder einem Gemisch beider Epoxide oxalkyliert,
b) anschließend das in Schritt a) erhaltene Oxalkylat der Formel (Ia)
b1) mit mindestens einem der Acylreste Z², Z³, Z⁴, Z⁵, Z⁶ und -OC-B-CO- zugrundeliegenden Carbonsäure oder einem reaktiven Derivat dieser Carbonsäure, vorzugsweise einem Anhydrid, in einer oder mehreren Stufen vollständig verestert oder teilverestert oder mit mehreren der vorstehend genannten Carbonsäuren oder deren reaktiven Derivaten mischverestert,
b2) und vorhandene Maleinsäurehalbestergruppen gegebenenfalls mit einem Sulfit oder schwefliger Säure umsetzt,
b3) gegebenenfalls die nach a) erhaltenen Oxalkylate mit einem dem Rest Z⁷ zugrundeliegenden Sulfatierungsmittel, vorzugsweise Schwefelsäure, Chlorsulfonsäure` Amidosulfonsäure oder Schwefeltrioxid, sulfatiert; und
c) im Falle, daß in b) ein Rest der Formel Z⁶ oder Z⁷ eingeführt wurde, gegebenenfalls mit einer dem Rest M zugrundeliegenden Base in das entsprechende Salz oder Oxalkylat überführt.

13. Verfahren nach Anspruch 12, dadurch gekennzeichnet, daß man
a) 1 bis 200 Mol, vorzugsweise 1 bis 50 Mol, insbesondere 5 bis 30 Mol, Ethylenoxid, Propylenoxid oder eine Kombination beider Epoxide pro Mol Alkylaminodialkylendiamin einsetzt,
b1) 1 bis 4 Mol mindestens einer der Acylresten Z², Z³, Z⁴, Z⁵, Z⁶ oder -OC-B-CO- zugrundeliegenden Carbonsäure(derivat) pro Mol des in Schritt a) erhaltenen Oxalkylats der Formel (Ia) einsetzt,
b2) 1 bis 1,5 Mol, vorzugsweise 1 bis 1,1 Mol, eines Sulfits oder schweflige Säure pro Mol Maleinsäurehalbestergruppe einsetzt,
b3) 1 bis 9 Mol, vorzugsweise 1 bis 4 Mol, eines dem Rest Z⁷ zugrundeliegenden Sulfatierungsmittels pro Mol des in Schritt a) erhaltenen Oxalkylats der Formel (Ia) einsetzt und
c) gegebenenfalls 1 bis 20 Mol, vorzugsweise 2 bis 10 Mol, einer dem Rest M zugrundeliegenden Base pro Mol der nach b2) oder b3) erhältlichen Verbindung einsetzt.

14. Verfahren nach Anspruch 12 oder 13, dadurch gekennzeichnet, daß man in Reaktionsschritt b) 2 bis 10, vorzugsweise 2 bis 5, Einheiten der Formel (Ia) durch Veresterung mit einer Dicarbonsäure der Formel HOOC-B-COOH oder mit einem dieser Dicarbonsäure zugrundeliegenden reaktiven Derivat im Molverhältnis Verbindung (Ia) : Dicarbonsäure(derivat) von 2:1 bis 10:9, vorzugsweise 2:1 bis 5:4, miteinander verknüpft.

15. Verfahren nach Anspruch 12 oder 13, dadurch gekennzeichnet, daß man in Reaktionsschritt b) ein Oxalkylat der Formel (Ia) mit einer oder mehreren der Reste Z², Z³, Z⁴, Z⁵, Z⁶ oder Z⁷ zugrundeliegenden Säure(derivate) teilverestert und anschließend 2 bis 10, vorzugsweise 2 bis 5, Einheiten der erhaltenen teilveresterten Oxalkylate durch Veresterung mit einer Dicarbonsäure der Formel HOOC-B-COOH oder mit einem dieser Dicarbonsäure zugrundeliegenden reaktiven Derivat im Molverhältnis teilverestertes Oxalkylat :
Dicarbonsäure(derivat) von 2:1 bis 10:9, vorzugsweise 2:1 bis 5:4, miteinander verknüpft.

16. Verwendung einer Verbindung nach mindestens einem der Ansprüche 1 bis 11 als oberflächenaktives Mittel.

17. Verwendung nach Anspruch 16 als Kupplungshilfs- und Präparationsmittel bei der Herstellung von Azofarbmitteln, vorzugsweise von Azopigmenten.

18. Verwendung nach Anspruch 16 als Dispergiermittel für die Herstellung von Feststoffdispersionen, vorzugsweise von anorganischen und organischen Pigmentdispersionen, zum Pigmentieren von wäßrigen Systemen, insbesondere Dispersionsanstrichfarben, wäßrige und wäßrig/alkoholische Flexodruckfarben und zum Pigmentieren von Viskose, Papiermassen und Leder sowie für die Herstellung von Gerbmitteldispersionen auf Metallsalzbasis.

19. Verwendung nach Anspruch 16 als Emulgiermittel bei der Herstellung von Flüssigemulsionen, bevorzugt Mineralöl- und Fettkörperemulsionen, Metallbearbeitungs- und Korrosionsschutzmitteln, Reinigungsverstärkern und Carrieremulsionen sowie für die Formulierung von Pflanzenschutz- und Schädlingsbekämpfungsmitteln.

20. Verwendung nach Anspruch 16 als Färbereihilfs-, Netz-, Egalisier-, Flotations-, Viskosezusatz- oder Viskoseveredlungsmittel, als Zusatzmittel für Spinnbäder sowie als Broschurhilfsmittel für Textilien und Leder.
